(19) Europäisches Patentamt
European Patent Office
Office européen des brevets

(11) EP 4 657 210 A1

(12) EUROPEAN PATENT APPLICATION
published in accordance with Art. 153(4) EPC

(43) Date of publication:
03.12.2025 Bulletin 2025/49

(51) International Patent Classification (IPC):
G06F 3/01 (2006.01)        A41D 19/00 (2006.01)
G01B 7/16 (2006.01)

(21) Application number: 24876081.1

(22) Date of filing: 14.06.2024

(52) Cooperative Patent Classification (CPC):
A41D 19/00; A61B 5/00; A61B 5/11; G01B 7/16;
G06F 3/01; G06V 40/20

(86) International application number:
PCT/CN2024/099228

(87) International publication number:
WO 2025/077256 (17.04.2025 Gazette 2025/16)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 12.10.2023 PCT/CN2023/124295

(71) Applicant: Shenzhen Shokz Co., Ltd.
Shenzhen, Guangdong 518108 (CN)

(72) Inventors:
• YUAN, Yongshuai
  Shenzhen, Guangdong 518108 (CN)
• DENG, Wenjun
  Shenzhen, Guangdong 518108 (CN)

(74) Representative: Wang, Bo
Panovision IP
Ebersberger Straße 3
85570 Markt Schwaben (DE)

(54) GLOVE SYSTEM

(57) A glove system (100), including a glove body (110) and a plurality of first strain sensors (120) and a plurality of second strain sensors (130) arranged on the glove body (110), when a user is wearing the glove body (110), each of the plurality of first strain sensors (120) is located at an interphalangeal joint of a finger of the user and is configured to measure deformation of the interphalangeal joint in a single degree of freedom, and each of the plurality of second strain sensors (130) is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger.

**100**

Glove body **110**

First strain sensor **120**

Second strain sensor **130**

Processor**140**

**FIG. 1**

## Description

### CROSS-REFERENCE TO RELATED APPLICATIONS

This application claims priority to International Application No. PCT/CN2023/124295, filed on October 12, 2023, the contents of which are hereby incorporated by reference.

### TECHNICAL FIELD

The present disclosure relates to the field of sensor technology, and in particular, to a glove system provided with a plurality of sensors and a method for recognizing a gesture based on the glove system.

### BACKGROUND

With the gradual maturation of AR/VR technology and the rise of the meta-universe concept, intelligent electronic devices set higher requirements for human-computer interaction technology. Flexible angle sensors can be conveniently integrated into wearable devices such as smart clothing and smart gloves, so as to realize accurate recognition and restoration of human movements, and are among the important underlying technologies of the meta-universe, which have received extensive attention and research. In order to realize accurate recognition and restoration of hand movements, there is a need to capture movements of the hand, especially the fingers, in a plurality of degrees of freedom.

Therefore, it is desirable to provide an electronic device that improves the accuracy of hand movement acquisition while controlling the cost.

### SUMMARY

One of the embodiments of the present disclosure provides a glove system, including: a glove body, and a plurality of first strain sensors and a plurality of second strain sensors arranged on the glove body, when a user is wearing the glove body, each of the plurality of first strain sensors is located at an interphalangeal joint of a finger of the user and is configured to measure deformation of the interphalangeal joint in a single degree of freedom, and each of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger.

In some embodiments, the plurality of first strain sensors and the plurality of second strain sensors collectively provide data reflecting deformation of the fingers of the user in no less than 15 degrees of freedom.

In some embodiments, the glove system further includes two inertial sensors, when the user is wearing the glove body, the two inertial sensors are respectively located on two sides of a wrist joint of the user and configured to jointly measure movement of the wrist joint in three degrees of freedom.

In some embodiments, the glove system further includes a third strain sensor disposed at the wrist joint of the user and an inertial sensor disposed outside the wrist joint.

In some embodiments, a first strain sensor of the plurality of first strain sensors includes an inductive sensor, and an inductance of the inductive sensor varies with the deformation of the interphalangeal joint.

In some embodiments, a second strain sensor of the plurality of second strain sensors includes a plurality of capacitive structures, capacitances of the plurality of capacitive structures vary with the deformation of the metacarpophalangeal joint in each degree of freedom.

In some embodiments, the second strain sensor includes: a flexible substrate; a first capacitive structure and a second capacitive structure, each of the first capacitive structure and the second capacitive structure includes a multilayer structure arranged on a same side surface of the flexible substrate in a thickness direction, and layers of each of the multilayer structures are stacked along the thickness direction.

In some embodiments, the glove body includes a fabric wrapped around each finger of the hand and a fabric disposed between neighboring fingers, an elastic modulus of the fabric between the neighboring fingers is less than an elastic modulus of the fabric wrapped around each finger.

In some embodiments, the fabric disposed between the neighboring fingers include at least one of a slit structure, an elastic fabric structure, or a pleated structure.

In some embodiments, the plurality of first strain sensors are arranged only at the interphalangeal joints of the fingers that are closest to the metacarpophalangeal joints.

In some embodiments, the glove system further including a processor configured to: read data associated with the plurality of first strain sensors and the plurality of second strain sensors; and determine a gesture of the user based on the data.

In some embodiments, the processor is configured to: output a gesture representation corresponding to the gesture of the user on a display interface; and output feedback information based on a distance between the gesture representation and a preset gesture.

In some embodiments, the processor is configured to: read a mapping relationship between gestures and voice packets; and play a voice message based on the gesture of the user and the mapping relationship.

In some embodiments, the processor is configured to: read a mapping relationship between gestures and instructions; and execute an instruction based on the gesture of the user and the mapping relationship.

One of the embodiments of the present disclosure provides a gesture reproduction method including: obtaining sensor data of a hand of a user based on a glove system, the glove system includes a glove body, and a plurality of first strain sensors and a plurality of second strain sensors arranged on the glove body, when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger; determining a gesture of the user based on the sensor data; outputting a gesture representation corresponding to the gesture on a display interface; and outputting feedback information based on a distance between the gesture representation and a preset gesture.

In some embodiments, the plurality of first strain sensors and the plurality of second strain sensors collectively provide data reflecting deformation of the fingers of the user in no less than 15 degrees of freedom.

One of the embodiments of the present disclosure provides a voice playback method including: obtaining sensor data of a hand of a user based on a glove system, the glove system includes a glove body, and a plurality of first strain sensors and a plurality of second strain sensors arranged on the glove body, when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger; determining a gesture of the user based on the sensor data; reading a mapping relationship between gestures and voice packets; and playing a voice message based on the gesture of the user and the mapping relationship.

In some embodiments, the plurality of first strain sensors and the plurality of second strain sensors collectively provide data reflecting deformation of the fingers of the user in no less than 15 degrees of freedom.

One of the embodiments of the present disclosure provides a gesture manipulation method including: obtaining sensor data of a hand of a user based on a glove system, the glove system includes a glove body, and a plurality of first strain sensors and a plurality of second strain sensors arranged on the glove body, when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger; determining a gesture of the user based on the sensor data; reading a mapping relationship between gestures and instructions; and executing an instruction based on the gesture of the user and the mapping relationship.

In some embodiments, the plurality of first strain sensors and the plurality of second strain sensors collectively provide data reflecting deformation of the fingers of the user in no less than 15 degrees of freedom.

BRIEF DESCRIPTION OF THE DRAWINGS

The present disclosure will be further illustrated with reference to a plurality of exemplary embodiments, described in detail using the accompanying drawings. These embodiments are not limiting, and in these embodiments, the same numbering denotes the same structure, where:

FIG. 1 is a schematic diagram illustrating an exemplary glove system according to some embodiments of the present disclosure;

FIG. 2A is a schematic diagram illustrating joints of a user's hand according to some embodiments of the present disclosure;

FIG. 2B is a schematic diagram illustrating joint positions of a user's hand according to some embodiments of the present disclosure;

FIG. 3 is a schematic diagram illustrating a coordinate system for hand movement according to some embodiments of the present disclosure;

FIG. 4 is a schematic diagram illustrating an exemplary glove system according to some embodiments of the present disclosure;

FIG. 5 is a schematic diagram illustrating a second strain sensor according to some embodiments of the present disclosure;

FIG. 6 is a schematic diagram illustrating a second strain sensor according to some further embodiments of the present disclosure;

FIG. 7A is a schematic diagram illustrating a distribution of four capacitive structures on a flexible substrate according to some embodiments of the present disclosure;

FIG. 7B is a schematic diagram illustrating a cross-sectional view of the structure shown in FIG. 7A along Y-axis;

FIG. 8A is a schematic diagram illustrating a sensor without deformation according to some embodiments of the present disclosure;

FIG. 8B is a schematic diagram illustrating deformation of the sensor as shown in FIG. 8A after being stretched or compressed along a long axis direction;

FIG. 9 is a schematic illustrating a side view of the sensor as shown in FIG. 8A after being bent around an axis parallel to a short-axis direction according to some embodiments of the present disclosure;

FIG. 10 is a schematic illustrating a side view of the sensor as shown in FIG. 8A after being bent and deformed around an axis parallel to the short-axis direction;

FIG. 11 is a schematic illustrating a cross-sectional view of a second strain sensor according to some embodiments of the present disclosure;

FIG. 12 is a flowchart illustrating a process performed by a processor in accordance with some embodiments of the present disclosure;

FIG. 13 is a processing flowchart illustrating a process performed by a processor according to some further embodiments of the present disclosure;

FIG. 14 is a flowchart illustrating a process performed by a processor according to some embodiments of the present disclosure;

FIG. 15 is a flowchart illustrating a process performed by a processor according to some embodiments of the present disclosure;

FIG. 16 is a flowchart illustrating a gesture reproduction method according to some embodiments of the present disclosure;

FIG. 17 is a flowchart illustrating a voice playback method according to some embodiments of the present disclosure; and

FIG. 18 is a flowchart illustrating a gesture manipulation method according to some embodiments of the present disclosure.

## DETAILED DESCRIPTION

In order to more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and it is possible for those skilled in the art to apply the present disclosure to other similar scenarios based on the accompanying drawings without creative labor. Unless obviously obtained from the context or the context illustrates otherwise, the same numeral in the drawings refers to the same structure or operation.

It will be understood that the term "system," "engine," "unit," "module," and/or "block" used herein are one method to distinguish different components, elements, parts, sections, or assembly of different levels in ascending order. However, the terms may be displaced by another expression if they achieve the same purpose.

As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Flowcharts are used in the present disclosure to illustrate operations performed by a system according to embodiments of the present disclosure. It should be understood that the preceding or subsequent operations are not necessarily performed precisely in sequence. On the contrary, the various steps can be processed in reverse order or simultaneously. Meanwhile, other operations may also be added to these processes, or a step or several steps can be removed from these processes.

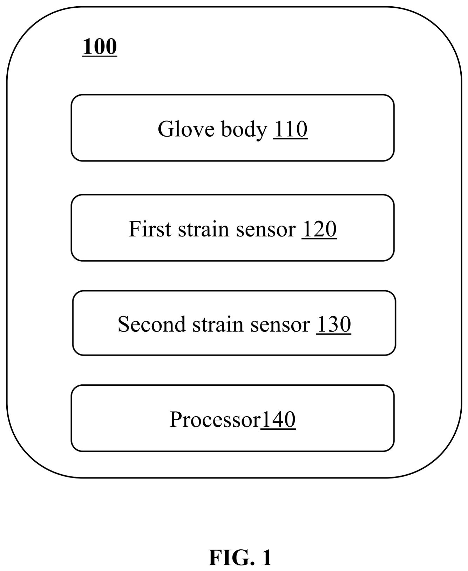

FIG. 1 is a schematic diagram illustrating an exemplary glove system according to some embodiments of the present disclosure. In some embodiments, as shown in FIG. 1, a glove system 100 may include a glove body 110, a plurality of strain sensors (including first strain sensors 120 and second strain sensors 130), and a processor 140. The plurality of strain sensors may be distributed at different positions on the glove body 110.

The glove system 100 refers to a system for capturing a user's hand movements (including finger movements). The glove system 100 may recognize hand movements via one or more devices, such as a motion capture glove, a sign language glove, a VR glove, a force feedback glove, a haptic feedback glove, or the like.

The glove body 110 refers to a wearable member for fitting with the hand. In some embodiments, to facilitate the fit of the glove body 110 to the hand joints (e.g., including interphalangeal joints, metacarpophalangeal joints, wrist joints, etc.), the glove body 110 has a structure adapted to the shape of the hand and extending along the hand joints. In some embodiments, the glove body 110 may be a flexible fabric that tends to deform following the fingers (e.g., bending deformation) when subjected to an external force. In some embodiments, the glove body 110 may provide support for the strain sensors to facilitate their arrangement. For example, the glove body 110 may include one or more layers of flexible structures (e.g., flexible fabric). The strain sensors may be fixed on a surface of a specific flexible structure layer by means of pasting, pressing or stitching.

The strain sensor refers to an electronic device that may convert deformation in the corresponding regions of the glove body 110 into electrical signals. In some embodiments, the strain sensors (e.g., the first strain sensor 120 and the second strain sensor 130) are arranged in one or more regions of the glove body 110 corresponding to the user's hand joints. When the user wears the glove body 110 to generate hand movement, the glove body 110 is deformed by an external force. The strain sensors may convert the deformation at the hand joints into electrical signals, and the electrical signals generated by the strain sensors may reflect the deformation direction, deformation magnitude, or the like in the corresponding regions.

In some embodiments, the strain sensors include a plurality of first strain sensors 120 and a plurality of second strain sensors 130.

Each first strain sensor 120 of the plurality of first strain sensors 120 may be configured to measure deformation in a single degree of freedom. In some embodiments, the first strain sensor 120 may be located at a position where the user's hand has fewer degrees of freedom of deformation, such as an interphalangeal joint, and be configured to measure deformation of the interphalangeal joint in the single degree of freedom. In some embodiments, the first strain sensor 120 includes a single-degree-of-freedom sensor, such as a single-axis sensor (e.g., an inductive sensor, a resistive bending sensor, or a capacitive stretching sensor).

Each second strain sensor 130 of the plurality of second strain sensors 130 may be configured to measure deformation in more than one degree of freedom. In some embodiments, the second strain sensor 130 may be located at a position of the user's hand having more degrees of freedom of deformation, such as a metacarpophalangeal joint, and be configured to measure deformation of the metacarpophalangeal joint in two degrees of freedom. In some embodiments, the second strain sensor 130 includes a multi-degree-of-freedom sensor, such as a multi-axis sensor (e.g., a capacitive multi-axis movement sensor).

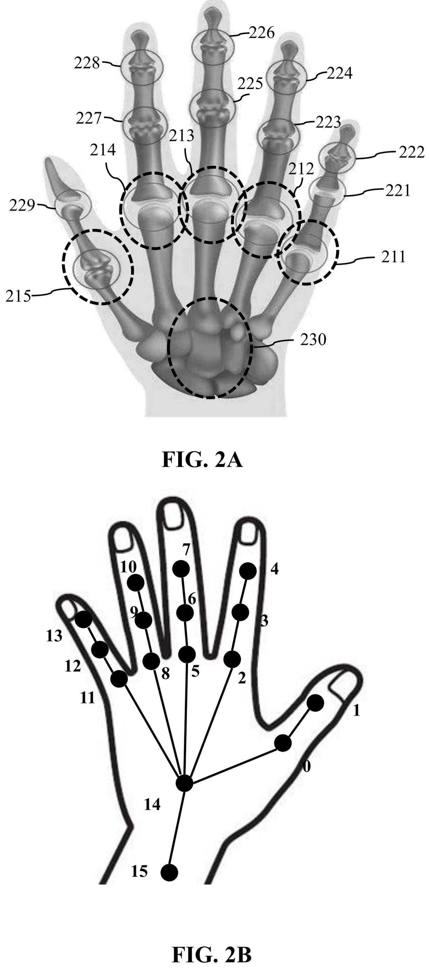

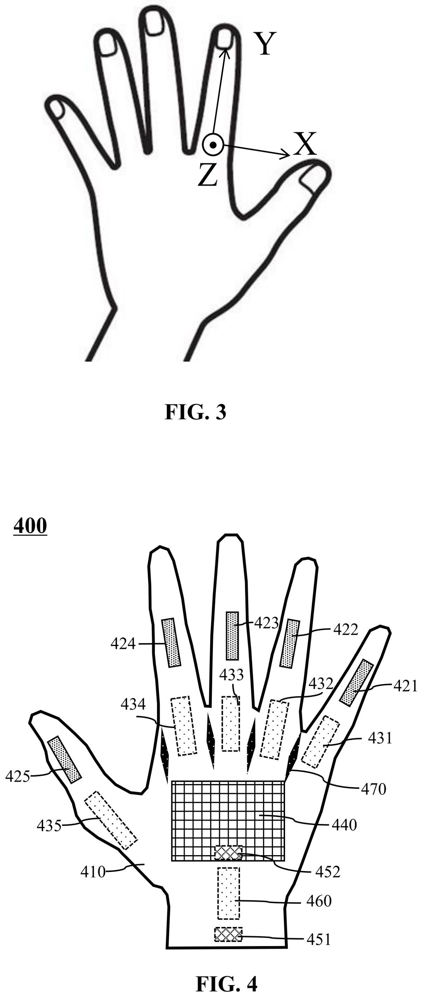

More details regarding the first strain sensor and the second strain sensor may be found in FIG. 4. More descriptions regarding the interphalangeal joints and the metacarpophalangeal joints may be found in FIG. 2A and FIG. 2B.

FIG. 2A is a schematic diagram illustrating joints of a user's hand according to some embodiments of the present disclosure. FIG. 2B is a schematic diagram illustrating joint positions of a user's hand according to some embodiments of the present disclosure.

As shown in FIG. 2A, metacarpophalangeal joints 211, 212, 213, 214, and 215 are joints between metacarpal capitula and proximal phalanges (first phalanges adjacent to metacarpal bones). Interphalangeal joints 221, 222, 223, 224, 225, 226, 227, 228, and 229 are joints between phalanges.

As shown in FIG. 2A and FIG. 2B, positions 0, 2, 5, 8, and 11 on the back of the hand (a dorsal side) correspond to the metacarpophalangeal joints 215, 214, 213, 212, and 211, respectively. Positions 1, 3, 6, 9, 12, 4, 7, 10, and 13 on the dorsal side of the hand correspond to the interphalangeal joints 229, 227, 225, 223, 221, 228, 226, 224, and 222, respectively.

In some embodiments, the first strain sensors 120 are arranged on the glove body 110 at the dorsal positions of the hand corresponding to the positions 1, 3, 6, 9, 12, 4, 7, 10, and 13, respectively. In some embodiments, the first strain sensor 120 may be arranged at positions on the front (a palm side) or side of the glove body 110 corresponding to the interphalangeal joints 229, 227, 225, 223, 221, 228, 226, 224, and 222. In some embodiments, the second strain sensors 130 are arranged on the glove body 110 at positions 0, 2, 5, 8, and 11 on the back of the hand, respectively.

Each first strain sensor 120 may measure deformation of one interphalangeal joint among the interphalangeal joints 229, 227, 225, 223, 221, 228, 226, 224, and 222 in a single degree of freedom. Each second strain sensor 130 may measure deformation of one metacarpophalangeal joint among metacarpophalangeal joints 215, 214, 213, 212, and 211 in two degrees of freedom.

In the present disclosure, degrees of freedom refer to the dimensions in which an object can move. To illustrate the degrees of freedom of a hand movement, a three-dimensional coordinate system is established for the hand, such as the coordinate system shown in FIG. 3. In the coordinate system, a metacarpophalangeal joint serves as the origin, finger extension direction as the Y-axis, and a direction perpendicular to the hand's plane as the Z-axis. The hand plane refers to a fitted plane of the palm region. For example, when the hand is placed flat on a table surface, either the table surface or a plane passing through the hand and parallel to the table surface may serve as the hand plane. The three-dimensional coordinate system may be used to describe the degrees of freedom of the movement of the hand. Specifically, in the three-dimensional coordinate system illustrated in FIG. 3, the rotation of the hand about the X-axis is defined as bending, the rotation of the hand about the Z-axis is defined as swinging, and the rotation of the hand about the Y-axis is defined as rotating. The interphalangeal joints (e.g., the interphalangeal joints 229, 227, 225, 223, 221, 228, 226, 224, and 222) may only perform bending and rotating. In other words, deformation of an interphalangeal joint corresponds to only one degree of freedom: bending. The metacarpophalangeal joints (e.g., the metacarpophalangeal joints 215, 214, 213, 212, and 211) may perform bending, swinging, and rotating. In other words, deformation of the metacarpophalangeal joint corresponds to two degrees of freedom: bending and swinging. As a result, each first strain sensor 120 may measure deformation in the bending degree of freedom for the interphalangeal joint among the interphalangeal joints 229, 227, 225, 223, 221, 228, 226, 224, and 222. When any interphalangeal joint undergoes bending movement, the first strain sensor 120 may measure deformation occurring at the corresponding interphalangeal joint on the glove body 110. Each second strain sensor 130 may measure deformation in the two degrees of freedom (bending and swinging degrees of freedom) for the metacarpophalangeal joint among the metacarpophalangeal joints 215, 214, 213, 212, and 211. When any metacarpophalangeal joint undergoes bending or swinging movement, the second strain sensor 130 may measure deformation occurring at the corresponding metacarpophalangeal joint on the glove body 110.

Continuing to refer to FIG. 2A and FIG. 2B, a hand has nine interphalangeal joints in total. The interphalangeal joints 228, 226, 224, and 222 are distal interphalangeal joints relatively distant from the metacarpals, and the interphalangeal joints 229, 227, 225, 223, and 221 are proximal interphalangeal joints relatively close to the metacarpals. In some embodiments, movement of the distal interphalangeal joints follows movement of previous joints (the proximal interphalangeal joints), and does not bend independently but bend together with the proximal interphalangeal joints. Therefore, the first strain sensors need not be arranged at the distal interphalangeal joints, but may be arranged only at the proximal interphalangeal joints. In some embodiments, deformation data at the distal interphalangeal joints may be generated by mapping deformation data at the corresponding proximal interphalangeal joints. The glove system 100 acquires deformation data for at least five proximal interphalangeal joints 229, 227, 225, 223, and 221 in a single degree of freedom via the plurality of first strain sensors 120, and collects deformation data for five metacarpophalangeal joints 215, 214, 213, 212, and 211 in two degrees of freedom via the plurality of second strain sensors 130. In total, the plurality of first strain sensors 120 and the plurality of second strain sensors 130 collectively provide deformation data in at least 15 degrees of freedom for the user's fingers.

By inferring or mapping deformation data at the distal interphalangeal joints using deformation data at the proximal interphalangeal joints, the first strain sensors need not be arranged at the distal interphalangeal joints. This reduces the count of strain sensors on the glove body, simplifies the glove system's structure to facilitate product cost control while maintaining deformation data acquisition accuracy.

In some embodiments, the glove system 100 may further include a processor 140. The processor 140 is configured to receive and process data collected by the strain sensors including the plurality of first strain sensors 120 and the plurality of second strain sensors 130. For example, the processor 140 may obtain deformation data from the strain sensors and analyze and process the data. For example, the processor 140 may obtain deformation magnitude and deformation angle of each finger joint through a mapping relationship (e.g., a mapping function or a trained machine learning model). In some embodiments, the processor 140 may directly process the data collected by the plurality of strain sensors, or the processor 140 may receive the data collected by the plurality of strain sensors and transmit the data to an external device for data processing. In some embodiments, the processor 140 may perform corresponding functions based on data processing results, such as gesture reproduction, voice playback, gesture manipulation, etc. as described below. In some embodiments, the processor 140 may interact with an external processing device based on data processing results. The external device includes an uplink such as a computer, a cell phone, a VR device, or the like. Interactions may include, for example, controlling a power state of the external device, adjusting volume, managing application processes of the external device (e.g., controlling movement of a game character), providing fitness status feedback, etc.

In some embodiments, the processor 140 may be a single server or a group of servers, and the group of servers may be centralized or distributed. In some embodiments, processor 140 may be local or remote. In some embodiments, the data collected by the strain sensor is transmitted to the processor 140 via a wire. In some embodiments, the processor 140 may be implemented on a cloud platform. For example, the cloud platform may include a private cloud, a public cloud, a hybrid cloud, a community cloud, a distributed cloud, an inter-cloud cloud, a multi-cloud, etc., or any combination thereof. In some embodiments, the processor 140 may include one or more processing chips, the processing chips analyzing and processing the received data. In some embodiments, the processor 140 is an independent processing device. In some embodiments, the processor 140 may be an external device or a part of the glove body 110. For example, the processor 140 may be integrated into the external device or the glove body 110.

Typically, in order to capture a multi-degree-of-freedom movement of the fingers, gyroscopes must be arranged at non-joint positions on the fingers to measure morphology across finger regions and deduce bending states; or, single-degree-of-freedom strain sensors (e.g., the first strain sensors 120) may be arranged at finger joints while gyroscopes are arranged at non-joint positions on the fingers. The measurement results of the two are combined to calculate the bending of the finger.. Some embodiments in the present disclosure control product costs, reduce subsequent data processing costs, and ensure measurement accuracy by using a combination of two degree of freedom strain sensors (e.g., the second strain sensors 130) and single degree of freedom strain sensors.

In some embodiments, the glove system 100 may further acquire wrist joint movement to more comprehensively identify hand movement. For example, the glove system 100 measures the wrist joint movement using a plurality of inertial sensors or using a combination of the strain sensors and the inertial sensors. More descriptions regarding acquisition of the wrist joint movement may be found in FIG. 4 and related descriptions thereof.

FIG. 4 is a schematic diagram illustrating an exemplary glove system according to some embodiments of the present disclosure.

As shown in FIG. 4, in some embodiments, the glove system 400 includes a glove body 410, a plurality of first strain sensors (including a first strain sensor 421, a first strain sensor 422, a first strain sensor 423, a first strain sensor 424, and a first strain sensor 425), a plurality of second strain sensors (including a second strain sensor 431, a second strain sensor 432, a second strain sensor 433, a second strain sensor 434, and a second strain sensor 435), and a processor 440. The first strain sensor is located in any one or more regions of the glove body 410 corresponding to the user's interphalangeal joints (e.g., the interphalangeal joints 229, 227, 225, 223, and 221). The second strain sensor is located in any one or more regions of the glove body 410 corresponding to the user's metacarpophalangeal joints (e.g., the metacarpophalangeal joints 211, 212, 213, 214, and 215). The processor 440 is configured to receive and process data collected by the strain sensors.

In some embodiments, the first strain sensor is a single-axis sensor that acquires deformation of an interphalangeal joint in a single degree of freedom (i.e., bending). In some embodiments, the first strain sensor includes various flexible bending sensors, such as an inductive sensor, a resistive sensor, a capacitive sensor, an optical fiber sensor, etc. Merely by way of example, the first strain sensor includes an inductive sensor. Inductance of the inductive sensor varies with deformation of the interphalangeal joint. Specifically, the inductive sensor deforms with movement of the interphalangeal joint, and the inductance of the inductive sensor changes in response to deformation of the inductive sensor. By acquiring electrical parameters related to the inductance of the inductive sensor and analyzing variations thereof, bending conditions of the inductive sensor (e.g., bending angle, bending direction, etc.) may be accurately obtained. For example, an electrical quantity of the inductance of the inductive sensor is proportional to a deformation amount of the inductive sensor. Thus, changes in the electrical quantity of the inductance of the inductive sensor may characterize deformation or bending degree of the interphalangeal joint.

Since the interphalangeal joint corresponds to only one bending degree of freedom, measuring movement of the interphalangeal joint using a single-axis sensor (e.g., the inductive sensor) achieves simple structure and low product cost.

In some embodiments, referring to FIG. 2A and FIG. 4, the first strain sensor is not arranged at a distal interphalangeal joint but only at a proximal interphalangeal joint. The deformation data at the distal interphalangeal joint may be mapped from the deformation data at the corresponding proximal interphalangeal joint. Referring to FIG. 2B and FIG. 4, the first strain sensors are arranged at the positions 1, 3, 6, 9, and 12 on the user's fingers. For example, the first strain sensor 425 is arranged at the position 1 on the thumb, where the position 1 corresponds to the proximal interphalangeal joint 229 on the thumb. As another example, the first strain sensor 424 is arranged at the position 3 on the index finger, where the position 3 corresponds to the proximal interphalangeal joint 227 on the index finger. As a further example, the first strain sensor 423 is arranged at the position 6 on the middle finger, where the position 6 corresponds to the proximal interphalangeal joint 225 on the middle finger.

Arranging the first strain sensors only at the interphalangeal joints closest to the metacarpophalangeal joints on each user's finger simplifies the structure of the glove system 400, reduces product costs, and maintains deformation data acquisition accuracy.

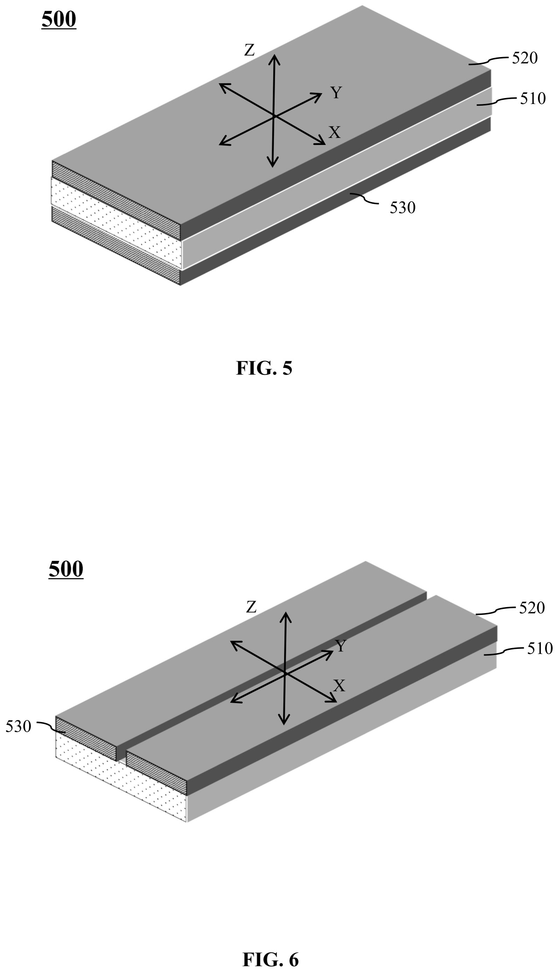

In some embodiments, the second strain sensor is a multi-axis sensor. The second strain sensor is configured to acquire deformation of the metacarpophalangeal joint in two degrees of freedom (i.e., bending and swinging). In some embodiments, the second strain sensor includes a plurality of flexible bending sensors, such as an inductive sensor, a resistive sensor, a capacitive sensor, an optical fiber sensor, etc. Merely by way of example, the second strain sensor includes a plurality of capacitive structures. Capacitances of the plurality of capacitive structures vary with deformation of the metacarpophalangeal joints in respective degrees of freedom. More descriptions regarding the second strain sensor including the plurality of capacitive structures may be found in FIGs. 5 to 8B and related descriptions thereof.

Referring to FIG. 5 and FIG. 6, in some embodiments, the second strain sensor 500 includes a flexible substrate 510, a first capacitive structure 520, and a second capacitive structure 530. In some embodiments, as shown in FIG. 5, the first capacitive structure 520 and the second capacitive structure 530 are distributed on opposite sides of the flexible substrate 510 along a thickness direction. A projection of the first capacitive structure 520 on the flexible substrate 510 at least partially overlaps a projection of the second capacitive structure 530 on the flexible substrate 510. In some embodiments, as shown in FIG. 6, the first capacitive structure 520 and the second capacitive structure 530 are distributed on a same side of the flexible substrate 510 along the thickness direction.

The flexible substrate 510 has flexible properties and easily deforms (e.g., bends) under external forces. In some embodiments, to facilitate attachment to human joints, the flexible substrate 510 may have a flat structure. In this case, the flexible substrate 510 has a thickness direction, which may be a Z-axis direction as shown in FIG. 5.

The capacitive structures (including the first capacitive structure 520 and the second capacitive structure 530) are configured to measure bending deformation of the flexible substrate 510. Specifically, each capacitive structure has a corresponding area. An electrical quantity (e.g., capacitance) of the capacitive structure is proportional to the area (or dimensional parameters related to the effective area, such as length/width of the capacitive structure or bending angle of the second strain sensor). The electrical quantities of the capacitive structure in different directions are proportional to the deformation amounts of the second strain sensor in different degrees of freedom, thereby reflecting deformation conditions (e.g., bending angle, bending direction). It should be understood that the second strain sensor may include, but is not limited to, a capacitive structure. For example, the second strain sensor may include a resistive structure or a composite capacitive-resistive structure. In some embodiments, each of the first capacitive structure 520 and the second capacitive structure 530 may include a multilayer structure arranged on a same side surface of the flexible substrate 510 in a thickness direction(e.g., the Z-axis direction shown in FIG. 5 or FIG. 6), and layers of each of the multilayer structures are stacked along the thickness direction. The multilayer structure refers to a configuration formed by stacking layered structures. "Each layer of each multilayer structure stacked along the thickness direction" refers to that each layer in the multilayer structure of the first capacitive structure 520 and the second capacitive structure 530 is arranged and stacked along the thickness direction of the flexible substrate 510. In some embodiments, the multilayer structure includes a second conductive layer, an intermediate layer, and a first conductive layer. In some embodiments, the multilayer structure includes a second conductive layer, a second intermediate layer, a third conductive layer, a first intermediate layer, and a first conductive layer. Specific layered structures included in the multilayer structure may be found below.

When different capacitive structures are arranged at different positions of the second strain sensor 500, respectively, the capacitive structures produce differentiated responses to deformation in different dimensions of the flexible substrate 510. When the flexible substrate 510 undergoes deformation in a specific dimension, the detection parameters generated by the capacitive structures have characteristics corresponding to the deformation in this dimension; when the flexible substrate 510 undergoes deformation in another dimension, the detection parameters generated by the capacitive structures again have characteristics corresponding to the deformation in that dimension. It may be understood that characteristics of detection parameters generated by the capacitive structures correspond one-to-one to deformation dimensions of the flexible substrate 510. Based on this, deformation of flexible substrate 510 may be identified according to detection parameters of each capacitive structure. In some embodiments, the processor (e.g., processor 140) may determine deformation of the flexible substrate 510 in at least two dimensions via a specific algorithm based on capacitance-related parameters of the capacitive structures (e.g., the first capacitive structure 520, the second capacitive structure 530). For example, deformation of the flexible substrate 510 in at least two dimensions may be determined using a machine learning model, the mapping relationship, or functional relationship. More descriptions of the deformation in at least two dimensions can be found below.

When the second strain sensor 500 (the flexible substrate 510) undergoes bending deformation, it causes changes in the physical shape of at least a portion of the capacitive structures (e.g., the first capacitive structure 520 and the second capacitive structure 530) disposed on the flexible substrate 510, thereby causing changes in their corresponding capacitances. For example, bending of the second strain sensor 500 causes a change in the area of the capacitive structure, thereby altering the capacitance of said capacitive structure. By acquiring capacitance or capacitance-related parameters of the capacitive structure and analyzing variations thereof, bending conditions of the second strain sensor 500 (e.g., bending angle, bending direction) may be accurately determined.

As shown in FIG. 5, in some embodiments, the first capacitive structure 520 and the second capacitive structure 530 are distributed on opposite sides of the flexible substrate 510 along the thickness direction. In some embodiments, the first capacitive structure 520 and the second capacitive structure 530 are symmetrically arranged along the thickness direction of the flexible substrate 510, where the projection of the first capacitive structure 520 on the flexible substrate 510 completely overlaps that of the second capacitive structure 530. When the first capacitive structure 520 and the second capacitive structure 530 are symmetrically arranged about the flexible substrate 510 along the thickness direction, both structures exhibit the same responses to some external disturbances (e.g., overall stretching or compression of the second strain sensor along a specific direction). By applying a differential processing algorithm to capacitance-related parameters of the capacitive structures, external interference effects on the second strain sensor 500 can be eliminated, thereby enhancing sensitivity in determining deformation of the flexible substrate 510 in at least two degrees of freedom.

In some embodiments, the flexible substrate 510 includes a short-axis direction and a long-axis direction that are perpendicular to the thickness direction, and the deformation on the plurality of degrees of freedom generated by the second strain transducer 500 (the flexible substrate 510) shown in FIG. 5 at least includes a bending deformation around an axis parallel to the short-axis direction and a tensile or compressive deformation along the long-axis direction. The short-axis direction may be the X-axis direction shown in FIG. 5; the long-axis direction may be the Y-axis direction shown in FIG. 5.

As shown in FIG. 5, when the second strain sensor 500 undergoes bending deformation about an axis parallel to the short-axis direction (i.e., the X-axis direction), the first capacitive structure 520 and the second capacitive structure 530 respectively experience tensile deformation and compressive deformation. For example, the first capacitive structure 520 becomes stretched due to bending while the second capacitive structure 530 becomes compressed due to bending, whereupon capacitances of the first capacitive structure 520 and the second capacitive structure 530 undergo opposing changes. In this case, the difference between capacitances of the first capacitive structure 520 and the second capacitive structure 530 may reflect bending direction and bending degree of the second strain sensor 500 about an axis parallel to the X-axis direction. Since the first capacitive structure 520 and the second capacitive structure 530 are stretched and compressed to substantially identical degrees, the sum of their capacitances may remain substantially constant.

When the second strain sensor 500 undergoes tensile or compressive deformation along the long-axis direction, the first capacitive structure 520 and the second capacitive structure 530 experience synchronous stretching or compression, resulting in synchronous changes in the capacitances of the first capacitive structure 520 and the second capacitive structure 530. In this case, the difference between the capacitances of the first capacitive structure 520 and the second capacitive structure 530 remains essentially constant (or close to zero). The sum of the capacitances of the first capacitive structure 520 and the second capacitive structure 530 may reflect a degree of tensile or compressive deformation of the second strain sensor 500 along the long-axis direction.

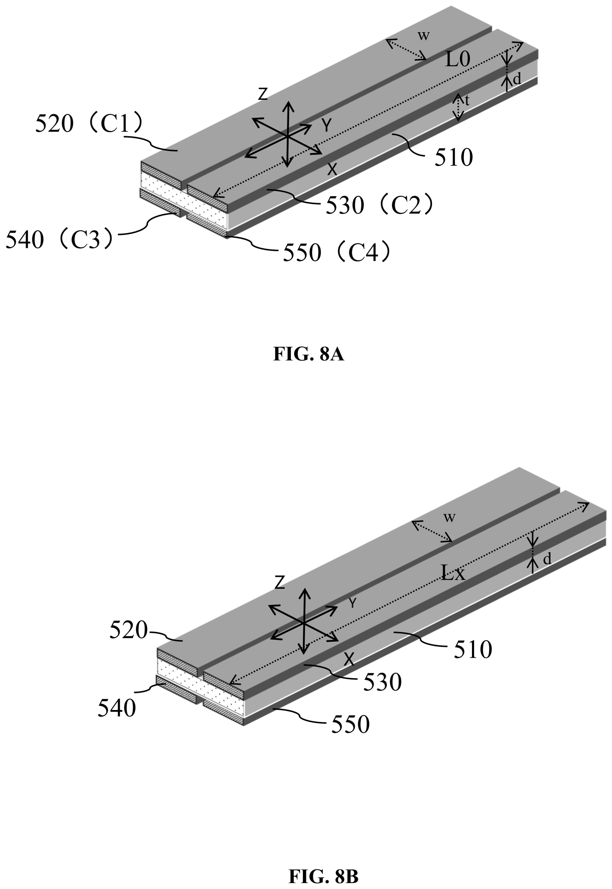

In some embodiments, by comparing the characteristics of the capacitance of the first capacitive structure 520 and the second capacitive structure 530 in the two degrees of freedom described above, for example, the relationship between the sum (difference) of the capacitances and the deformation of the flexible substrate 510, mutual interference between the deformations of the two degrees of freedom can be avoided, and the deformation of the flexible substrate 510 in the two degrees of freedom can be effectively distinguished. For example, when the second strain sensor 500 is stretched or compressed as a whole, the first capacitive structure 520 and the second capacitive structure 530 will be stretched or compressed synchronously, and the synchronized stretching or compression of the first capacitive structure 520 and the second capacitive structure 530 is regarded as a common-mode interference, and at this time, differential processing of the signals (e.g., capacitance) can exclude such common-mode interference, so that the second strain sensor 500 is insensitive to its own tensile or compressive deformation, and is only sensitive to the bending deformation around the axis parallel to the short axis direction (i.e., the X-axis direction), which enables the second strain sensor 500 to accurately detect the bending deformation of this degree of freedom. That is, the difference between capacitances of the first capacitive structure 520 and the second capacitive structure 530 primarily reflects bending deformation of the flexible substrate 510 about an axis parallel to the short-axis direction (i.e., the X-axis direction). Similarly, the sum of capacitances of the first capacitive structure 520 and the second capacitive structure 530 primarily reflects tensile or compressive deformation of the flexible substrate 510 along the long-axis direction. More description regarding the detection principle for tensile or compressive deformation along the long-axis direction is similar to that of the second strain transducer 500 with four capacitive structures, as may be illustrated by reference to FIGS. 8A to 10 and related descriptions thereof.

As shown in FIG. 6, in some embodiments, the flexible substrate 510 includes a long-axis direction perpendicular to the thickness direction. The first capacitive structure 520 and the second capacitive structure 530 are distributed on the same side of the flexible substrate 510 along the thickness direction, arranged side-by-side and both extending along the long-axis direction of the flexible substrate 510.

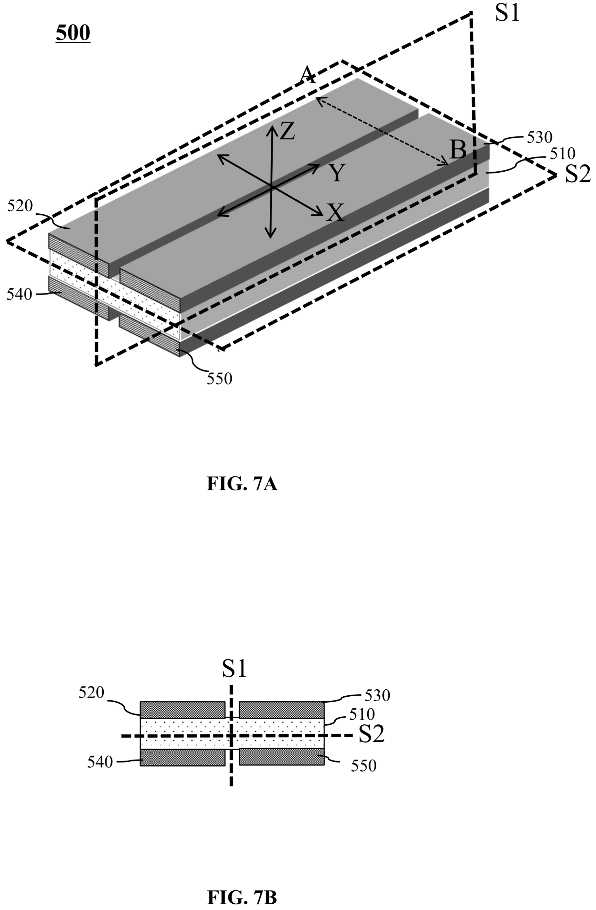

In some embodiments, the first capacitive structure 520 and the second capacitive structure 530 are symmetrically arranged about a mid-section parallel to the plane formed by thickness direction and long-axis direction on the flexible substrate 510. When the first capacitive structure 520 and the second capacitive structure 530 are symmetrically arranged about the flexible substrate 510's mid-section, both structures exhibit the same responses to some external disturbances (e.g., overall stretching or compression of the second strain sensor 500 along a specific direction). The processor can eliminate these disturbances' effects on the second strain sensor 500 by applying a differential algorithm to capacitance-related parameters of the capacitive structures (e.g., the first capacitive structure 520 and the second capacitive structure 530), thereby enhancing sensitivity in determining deformation of the flexible substrate 510 in at least two degrees of freedom. More descriptions of the mid-section can be found in FIG. 7 A and FIG. 7 B and their related descriptions.

In some embodiments, the second strain sensor 500 (the flexible substrate 510) shown in FIG. 6 produces a deformation in degrees of freedom that includes at least a bending deformation about an axis parallel to the thickness direction and a stretching or compression deformation along the long-axis direction.

As shown in FIG. 6, when the second strain sensor 500 is bent and deformed about an axis parallel to the thickness direction (i.e., the Z-axis direction), the first capacitive structure 520 and the second capacitive structure 530 undergo tensile deformation and compressive deformation, respectively. For example, the first capacitive structure 520 is stretched by bending, and the second capacitive structure 530 is compressed by bending. At this time, the capacitances of the first capacitive structure 520 and the second capacitive structure 530 change in opposite directions. In this case, a difference between the capacitances of the first capacitive structure 520 and the second capacitive structure 530 may reflect a bending direction and a bending degree of the second strain sensor 500 about an axis parallel to the Z-axis direction. Since the first capacitive structure 520 and the second capacitive structure 530 are stretched and compressed to substantially a same degree, the sum of the capacitances of the first capacitive structure 520 and the second capacitive structure 530 may remain substantially constant.

When the second strain transducer 500 shown in FIG. 6 is stretched or compressed along the long-axis direction, the first capacitive structure 520 and the second capacitive structure 530 are synchronously stretched or compressed. At this time, the capacitances of the first capacitive structure 520 and the second capacitive structure 530 change synchronously. In this case, the difference between the capacitance of the first capacitive structure 520 and the second capacitive structure 530 remains essentially constant (or close to zero), while the sum of the two may reflect the degree of stretching or compression of the second strain sensor 500 along the long-axis direction.

In some embodiments, by comparing the features of the capacitances of the first capacitive structure 520 and the second capacitive structure 530 in the two degrees of freedom (for example, a relationship between the sum (or a difference) of the capacitances and a deformation of the flexible substrate 510), mutual interference between deformations of the two degrees of freedom may be avoided, and the deformation of the flexible substrate 510 in the two degrees of freedom may be effectively distinguished. For example, when the second strain sensor 500 is entirely stretched or compressed, the first capacitive structure 520 and the second capacitive structure 530 are synchronously stretched or compressed. The synchronous stretching or compression of the first capacitive structure 520 and the second capacitive structure 530 may be regarded as common-mode interference. At this time, differential processing of signals (e.g., the capacitances) may exclude such common-mode interference, so that the second strain sensor 500 is insensitive to its own stretching or compression deformation, and is only sensitive to bending deformation about an axis parallel to the thickness direction (i.e., the Z-axis direction), enabling the second strain sensor 500 to accurately detect bending deformation in the degree of freedom. That is to say, the difference between the capacitances of the first capacitive structure 520 and the second capacitive structure 530 mainly reflects bending deformation of the flexible substrate 510 about an axis parallel to the thickness direction (i.e., the Z-axis direction). Similarly, the sum of the capacitances of the first capacitive structure 520 and the second capacitive structure 530 mainly reflects stretching or compression deformation of the flexible substrate 510 along the long-axis direction. More details about detection principles for stretching or compression deformation along the long-axis direction are similar to detection principles of the second strain sensor 500 having four capacitive structures. More descriptions may refer to FIG. 8A to FIG. 10 and related descriptions thereof.

FIG. 7A is a schematic diagram of a distribution of four capacitive structures on a flexible substrate according to some embodiments of the present disclosure; FIG. 7B is a schematic diagram of a cross-sectional structure viewed along the Y-axis as shown in FIG. 7A.

As shown in FIG. 7A and FIG. 7B, in some embodiments, compared with FIG. 6, the second strain sensor 500 further includes a third capacitive structure 540 and a fourth capacitive structure 550. The first capacitive structure 520 and the second capacitive structure 530 are arranged side by side on one side surface of the flexible substrate 510 along a thickness direction and both extend along a long-axis direction. The third capacitive structure 540 and the fourth capacitive structure 550 are arranged side by side on another side surface of the flexible substrate 510 along the thickness direction and both extend along the long-axis direction.

In some embodiments, a processor reads capacitance-related parameters of the first capacitive structure 520, the second capacitive structure 530, the third capacitive structure 540, and the fourth capacitive structure 550 respectively, and determines deformation of the flexible substrate 510 in at least two degrees of freedom based on the parameters.

In some embodiments, deformation in degrees of freedom generated by the second strain sensor 500 (the flexible substrate 510) shown in FIG. 7A at least includes: bending deformation about an axis parallel to the thickness direction, bending deformation about an axis parallel to a short-axis direction, and stretching or compression deformation along the long-axis direction of the flexible substrate 510. For illustrative purposes, the following describes the capacitance-related parameters of each capacitive structure under different degrees of freedom deformation with reference to FIG. 8A, FIG. 8B, FIG. 9, and FIG. 10 respectively.

In some embodiments, the capacitance-related parameters include: C1, C2, C3, C4, Ctotal, dCx, and dCz, where Ctotal=C1+C2+C3+C4, dCx=(C1+C2)-(C3+C4) after composite differential operation, and dCz=(C1+C3)-(C2+C4) after composite differential operation. C1, C2, C3, and C4 represent capacitances of the first capacitive structure 520, the second capacitive structure 530, the third capacitive structure 540, and the fourth capacitive structure 550, respectively.

In some embodiments, the first capacitive structure 520 and the second capacitive structure 530 are symmetrically arranged about a first mid-section S1. The third capacitive structure 540 and the fourth capacitive structure 550 are symmetrically arranged about the first mid-section S1. The first mid-section S1 represents a cross-section of the flexible substrate 510 parallel to a plane formed by the thickness direction and the long-axis direction. The first capacitive structure 520 and the third capacitive structure 540 are symmetrically arranged about a second mid-section S2 of the flexible substrate 510. The second capacitive structure 530 and the fourth capacitive structure 550 are symmetrically arranged about the second mid-section S2. The second mid-section S2 represents a cross-section of the flexible substrate 510 parallel to a plane formed by the long-axis direction and a short-axis direction.

By arranging four symmetric capacitive structures on the second strain sensor 500 and performing composite differential operations, common-mode interference can be effectively eliminated during the process of identifying bending deformation of the sensor. Merely by way of example, when the second strain sensor 500 undergoes overall stretching or compression deformation, the first capacitive structure 520, second capacitive structure 530, third capacitive structure 540 and fourth capacitive structure 550 will be synchronously stretched or compressed. The stretching or compression of the four capacitive structures may be taken as common-mode interference, the values of dCx and dCz obtained through composite differential processing of the signals remain unchanged, such that the common-mode interference can be effectively eliminated. This makes the second strain sensor 500 insensitive to its own stretching or compression deformation while remaining sensitive only to its bending deformation, thereby improving the accuracy of the second strain sensor 500.

FIG. 8A is a schematic diagram of the structure of the sensor without deformation according to some embodiments of the present disclosure; FIG. 8B is a schematic diagram of the deformation of the sensor after being stretched or compressed along the long-axis direction as shown in FIG. 8A.

In some embodiments, when the first capacitive structure 520, the second capacitive structure 530, the third capacitive structure 540, and the fourth capacitive structure 550 are all capacitive structures, the second strain sensor 500 may identify stretching or compression deformation along the long-axis direction of the flexible substrate 11 based on the capacitance-related parameters.

In some embodiments, when the second strain sensor 500 shown in FIG. 8A is undeformed, the capacitances of the four capacitive structures may be obtained according to the following Equation (1). Merely for illustrative purposes, the four capacitive structures of the second strain sensor 500 are considered completely the same and symmetrically arranged about the flexible substrate 510 (i.e., the first mid-section S1 and the second mid-section S2).

$$C1 = C2 = C3 = C4 = \varepsilon_0 \varepsilon \frac{A}{d} = \varepsilon_0 \varepsilon \frac{L0w}{d} \quad (1)$$

where $\varepsilon_0$ represents the vacuum permittivity; $\varepsilon$ represents the relative permittivity of each of the four capacitive structures (i.e., an intermediate layer, as described below); d represents the thickness of the dielectric layer of the each capacitive structure; A represents the area of each capacitive structure on the plane formed by the long-axis direction and short-axis direction; L0 represents the initial length of each capacitive structure along the long-axis direction of the flexible substrate 510; and w represents the width of the each capacitive structure along the short-axis direction of the flexible substrate 510.

In some embodiments, as shown in FIG. 8B, when the second strain sensor 500 is stretched or compressed along the long-axis direction (i.e., the Y-axis direction), the initial length L0 of each of the four capacitive structures is elongated or compressed to Lx. Considering that the thickness variation of the each capacitive structure of the four capacitive structures is negligible, for simplicity, the thickness of the each capacitive structure of the four capacitive structure is assumed to remain unchanged. According to Equation (1), the capacitance change of the four capacitive structures may be expressed by the following Equation (2).

$$C1 = C2 = C3 = C4 = \varepsilon_0 \varepsilon \frac{Lxw}{d}. \quad (2)$$

At this time, Ctotal, dCx, dCz are determined by the following Equations (3), (4), and (5), respectively.

$$Ctotal = C1 + C2 + C3 + C4 = 4\varepsilon_0 \varepsilon \frac{Lxw}{d} \quad (3)$$

$$dCx = (C1 + C2) - (C3 + C4) = 0 \quad (4)$$

$$dCz = (C1 + C3) - (C2 + C4) = 0. \quad (5)$$

When the second strain sensor 500 is stretched or compressed along its long-axis direction, the four capacitive structures are synchronously stretched or compressed, and the capacitances of the four capacitive structures synchronously change. In this case, the sum of the capacitances of the four capacitive structures may reflect a degree of stretching or compression of the second strain sensor 500 (the flexible substrate 510) along the long-axis direction. According to Equations (2) and (3), Ctotal is only related to length Lx after a change of the capacitive structures, and Lx may be used to represent deformation of the second strain sensor 500 along its long-axis direction. Thus, Ctotal may be used to reflect deformation of the second strain sensor 500 (the flexible substrate 510) along its long-axis direction.

According to Equation (4), dCx reflects a difference between a sum of a capacitance C1 of the first capacitive structure 520 and a capacitance C2 of the second capacitive structure 530, and a sum of a capacitance C3 of the third capacitive structure 540 and a capacitance C4 of the fourth capacitive structure 550. Referring to a structural distribution of the four capacitive structures in FIG. 8B, the sum of the capacitance C1 of the first capacitive structure 520 and the capacitance C2 of the second capacitive structure 530, i.e., C1+C2, may reflect bending deformation of an upper side surface of the flexible substrate 510 in a thickness direction. The sum of the capacitance C3 of the third capacitive structure 540 and the capacitance C4 of the fourth capacitive structure 550, i.e., C3+C4, may reflect bending deformation of a lower side surface of the flexible substrate 510 in the thickness direction. dCx is used to reflect a difference between bending deformations of upper and lower side surfaces of the flexible substrate 510 (i.e., upper and lower side surfaces along the thickness direction) during deformation of the flexible substrate 510. dCx is related to bending deformation about an axis parallel to a short-axis direction (i.e., X-axis direction), and stretching or compression deformation along a long-axis direction (i.e., Y-axis direction) does not cause a deformation difference between the upper and lower side surfaces of the flexible substrate 510, so dCx is a constant 0.

Similarly, according to Equation (5), dCz may be used to reflect a difference between bending deformations of two side surfaces of the flexible substrate 510 along the short-axis direction during deformation of the flexible substrate 510. dCz is only related to bending deformation about an axis parallel to the thickness direction (i.e., Z-axis direction), and stretching or compression deformation along the long-axis direction (i.e., Y-axis direction) does not cause a deformation difference between the two side surfaces of the flexible substrate 510 along the short-axis direction, so dCz is also a constant 0.

In some embodiments, an output parameter Ctotal of the second strain sensor 500 stretched or compressed along the Y-axis changes linearly and proportionally with a stretched length or a compressed length Lx, and has extremely high linearity, while dCz and dCx are both 0 and are not affected by the stretched length Lx. Therefore, the second strain sensor 500 may identify stretching or compression deformation along the long-axis direction of the flexible substrate 510 based on the parameter Ctotal related to the capacitances.

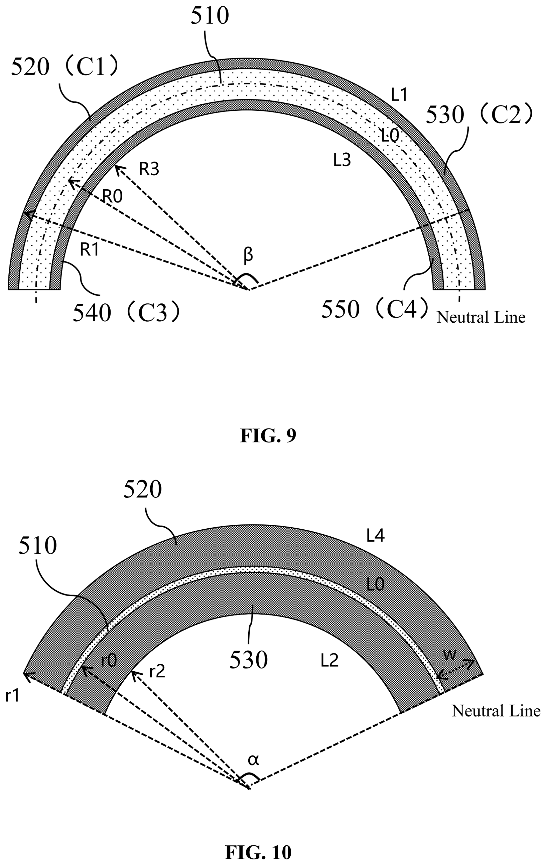

FIG. 9 is a schematic illustrating a side view of the sensor as shown in FIG. 8A after being bent around an axis parallel to a short-axis direction according to some embodiments of the present disclosure.

In some embodiments, when the first capacitive structure 520, the second capacitive structure 530, the third capacitive structure 540, and the fourth capacitive structure 550 are all capacitive structures, the second strain sensor 500 may identify bending deformation about the axis parallel to the short-axis direction of the flexible substrate 510 based on the parameter dCx related to capacitances.

As shown in FIG. 8A and FIG. 9, after the second strain sensor 500 is bent about an axis parallel to the short-axis direction (i.e., the X-axis direction), for ease of description, the bent second strain sensor 500 may be approximated as an arc. The first capacitive structure 520 and the second capacitive structure 530 are bent and elongated to L1. The third capacitive structure 540 and the fourth capacitive structure 550 are bent and compressed to L3. Considering that a thickness change of each of the four capacitive structures is very small, for ease of description, the thickness of each of the four capacitive structures may be approximated as unchanged. Then, according to Equation (1), the capacitance C1 of the first capacitive structure 520 and the capacitance C2 of the second capacitive structure 530 change according to Equation (6) below. The capacitance C3 of the third capacitive structure 540 and the capacitance C4 of the fourth capacitive structure 550 change according to Equation (7) below.

$$C1 = C2 = \varepsilon_0 \varepsilon \frac{L1w}{d} = \varepsilon_0 \varepsilon \frac{R1\beta w}{d} = \varepsilon_0 \varepsilon \frac{(R0+t/2)\beta w}{d} \quad (6)$$

$$C3 = C4 = \varepsilon_0 \varepsilon \frac{L3w}{d} = \varepsilon_0 \varepsilon \frac{R3\beta w}{d} = \varepsilon_0 \varepsilon \frac{(R0-t/2)\beta w}{d}. \quad (7)$$

Ctotal, dCx, and dCz are determined by the following Equations (8), (9), and (10), respectively.

$$Ctotal = C1 + C2 + C3 + C4 = 4\varepsilon_0 \varepsilon \frac{L0w}{d} \quad (8)$$

$$dCx = (C1 + C2) - (C3 + C4) = \frac{2\varepsilon_0 \varepsilon wt}{d}\beta \quad (9)$$

$$dCz = (C1 + C3) - (C2 + C4) = 0, \quad (10)$$

where R0 represents a radius of a circular arc of bending deformation of the flexible substrate 510; R1 represents a radius of a circular arc of bending deformation of the first capacitive structure 520 and the second capacitive structure 530; R2 represents a radius of a circular arc of bending deformation of the third capacitive structure 540 and the fourth capacitive structure 550; t represents a thickness of the second strain sensor 500 as shown in FIG. 8A; L1 represents a length of the circular arc of bending deformation of the first capacitive structure 520 and the second capacitive structure 530; and L3 represents a length of the circular arc of bending deformation of the third capacitive structure 540 and the fourth capacitive structure 550.

When the second strain sensor 500 is bent about an axis parallel to the short-axis direction, for example, the first capacitive structure 520 and the second capacitive structure 530 undergo bending stretching, and the third capacitive structure 540 and the fourth capacitive structure 550 undergo bending compression. At this point, the sum of the capacitance C1 of the first capacitive structure 520 and the capacitance C2 of the second capacitive structure 530, i.e., C1+C2, and the sum of the capacitance C3 of the third capacitive structure 540 and the capacitance C4 of the fourth capacitive structure 550, i.e., C3+C4, have opposite change relationships. The Ctotal obtained by adding capacitances of the four capacitive structures may offset change trends of C1+C2 and C3+C4, i.e., at this point Ctotal is a constant as shown in Equation (8). Thus, Ctotal cannot be used to reflect bending deformation of the second strain sensor 500 about the axis parallel to the short-axis direction.

In some embodiments, after the second strain sensor 500 is bent about an axis parallel to the short-axis direction (i.e., X-axis direction), an output parameter dCx changes linearly and proportionally with a bending angle $\beta$ and has extremely high linearity, while dCz and Ctotal are both constants and are unaffected by the bending angle $\beta$. Therefore, the second strain sensor 500 may identify bending deformation about the axis parallel to the short-axis direction based on a parameter dCx related to capacitances.

It should be noted that, in addition to the case of the four capacitive structures described above, the second strain sensor 500 may have three or more capacitive structures distributed on upper and lower side surfaces of the flexible substrate 510 along the thickness direction, all extending along the long axis direction. As long as deformations in different dimensions may be characterized by parameters related to capacitances of the capacitive structures, deformations in a plurality of dimensions may be identified. Merely by way of example, when the second strain sensor 500 has three capacitive structures, two of the three capacitive structures are distributed on an upper side of the flexible substrate, and one is distributed on a lower side of the flexible substrate. At this point, the capacitance-related parameters of the three capacitive structures are also related to bending deformation about the X-axis (or the Z-axis). At this point, bending deformation of the second strain sensor 500 about the X-axis (or the Z-axis) may also be obtained by combining the capacitance-related parameters to respectively generated by the three capacitive structures.

FIG. 10 is a schematic illustrating a side view of the sensor as shown in FIG. 8A after being bent and deformed around an axis parallel to the short-axis direction.

In some embodiments, when the first capacitive structure 520, the second capacitive structure 530, the third capacitive structure 540, and the fourth capacitive structure 550 are capacitive structures, the second strain sensor 500 may identify bending deformation about an axis parallel to the thickness direction of the flexible substrate 510 based on the parameter dCz related to capacitances.

As shown in FIGs. 8A and 10, after the second strain sensor 500 is bent about an axis parallel to the thickness direction (i.e., Z-axis direction), for ease of description, the bent second strain sensor 500 may be approximated as a circular arc. The first capacitive structure 520 and the third capacitive structure 540 (not shown in FIG. 10) are bent and elongated to L4. The second capacitive structure 530 and the fourth capacitive structure 550 (not shown in FIG. 10) are bent and compressed to L2. Considering that thickness change of each of the four capacitive structures is very small, for ease of description, the thickness of each of the four capacitive structures may be approximated as unchanged. Then, according to Equation (1), the capacitance C1 of the first capacitive structure 520 and the capacitance C3 of the third capacitive structure 540 change according to Equation (11) below, and the capacitance C2 of the second capacitive structure 530 and the capacitance C4 of the fourth capacitive structure 550 change according to Equation (12) below.

$$C1 = C3 = \varepsilon_0 \varepsilon \frac{(L0+L4)\ w}{2d} = \varepsilon_0 \varepsilon \frac{(r0\alpha + r1\alpha)w}{2d} = \varepsilon_0 \varepsilon \frac{(2r0+w)\alpha w}{2d} \quad (11)$$

$$C2 = C4 = \varepsilon_0 \varepsilon \frac{(L0+L2)\ w}{2d} = \varepsilon_0 \varepsilon \frac{(r0\alpha + r2\alpha)w}{2d} = \varepsilon_0 \varepsilon \frac{(2r0-w)\alpha w}{2d} \quad (12)$$

Ctotal, dCx, and dCz are determined by Equations (13), (14), and (15) below, respectively.

$$Ctotal = C1 + C2 + C3 + C4 = 4\varepsilon_0\varepsilon\frac{L0w}{d} \quad （13）$$

$$dCx = （C1 + C2） - （C3 + C4） = 0 \quad （14）$$

$$dCz = （C1 + C3） - （C2 + C4） = \frac{2\varepsilon_0\varepsilon w^2}{d}\alpha \quad （15）$$

where r0 represents a radius of a circular arc of bending deformation of the flexible substrate 510; r1 represents a radius of a circular arc of bending deformation of the first capacitive structure 520 and the third capacitive structure 540; r2 represents a radius of a circular arc of bending deformation of the second capacitive structure 530 and the fourth capacitive structure 550; L2 represents a length of the circular arc of bending deformation of the first capacitive structure 520 and the third capacitive structure 540; and L4 represents a length of the circular arc of bending deformation of the second capacitive structure 530 and the fourth capacitive structure 550.

In some embodiments, after the second strain sensor 500 is bent about the axis parallel to the thickness direction (i.e., Z-axis direction), the output parameter dCz changes linearly and proportionally with a bending angle $\alpha$ and has extremely high linearity, while dCx and Ctotal are both constants and are unaffected by the bending angle $\alpha$. Therefore, the second strain sensor 500 may identify bending deformation about the axis parallel to the thickness direction based on the parameter dCz related to capacitances.

In some embodiments, when the first capacitive structure 520, the second capacitive structure 530, the third capacitive structure 540, and the fourth capacitive structure 550 are resistive structures, a processor may determine deformation of the flexible substrate 510 in at least two dimensions based on resistance-related parameters of the four capacitive structures. Merely by way of example, resistances of the four capacitive structures are respectively: $R_1 = R_2 = R_3 = R_4 = \rho\, d/A$. By substituting areas of the each capacitive structure after deformation of the second strain sensor 500 into the equation and performing differential operations, results similar to capacitance-related parameters may be obtained, enabling the processor to determine bending deformation of the second strain sensor 500 about the axis parallel to the thickness direction, bending deformation about the axis parallel to the short-axis direction, and stretching or compression deformation along the long-axis direction of the flexible substrate. Here, $\rho$ represents a resistivity of each of the four capacitive structures (i.e., an intermediate layer, see description below).

In some embodiments, the processor may identify composite deformation where deformations in any dimension among bending deformation of the second strain sensor 500 about the axis parallel to the thickness direction, bending deformation about the axis parallel to the short-axis direction, and stretching or compression deformation along the long-axis direction of the flexible substrate 510 coexist simultaneously. For example, the processor may simultaneously obtain changes in the parameters Ctotal, dCx, and dCz related to capacitances, thereby simultaneously restoring deformation components of different dimensions to restore actual bending deformation of the second strain sensor 500.

Thus, embodiments of the present disclosure can measure deformations in a plurality of degrees of freedom (e.g., including three dimensions: bending about the X-axis, bending about the Z-axis, and stretching along the Y-axis) via a single sensor, achieving detection of movement information in a plurality of dimensions. This improves detection accuracy while facilitating simplified manufacturing processes and miniaturized designs.

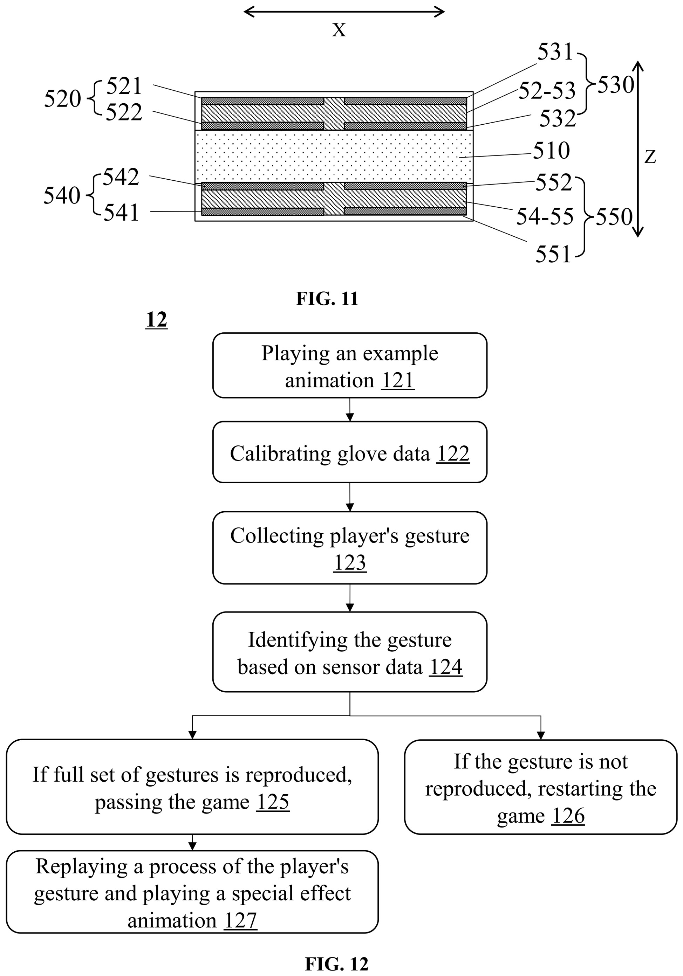

FIG. 11 is a schematic illustrating a cross-sectional view of a second strain sensor according to some embodiments of the present disclosure.

As shown in FIG. 11, in some embodiments, each capacitive structure includes a second conductive layer, an intermediate layer, and a first conductive layer stacked sequentially away from the flexible substrate 510 along a thickness direction. For example, the first capacitive structure 520 includes a second conductive layer 522, an intermediate layer 52-53, and a first conductive layer 521 stacked sequentially away from the flexible substrate 510. The second capacitive structure 530 includes a second conductive layer 532, the intermediate layer 52-53, and a first conductive layer 531 stacked sequentially away from the flexible substrate 510. The third capacitive structure 540 includes a second conductive layer 542, an intermediate layer 54-55, and a first conductive layer 541 stacked sequentially away from the flexible substrate 510. The fourth capacitive structure 550 includes a second conductive layer 552, the intermediate layer 54-55, and a first conductive layer 551 stacked sequentially away from the flexible substrate 510. In some embodiments, the intermediate layer is disposed between the first conductive layer and the second conductive layer.

In some embodiments, the first conductive layer and the second conductive layer of the each capacitive structure include an elastic conductive material. The elastic conductive material provides conductivity to the first conductive layer and the second conductive layer and allows them to return to an initial shape when an external force is removed. In some embodiments, the elastic conductive material includes, but is not limited to, a conductive adhesive film, a conductive ink, a conductive polymer material, a conductive gel, a liquid metal, etc. In some embodiments, the first conductive layer and the second conductive layer include a conductive adhesive film formed by mixing conductive particles with a polymer material.

In some embodiments, the polymer material includes, but is not limited to, a silicone, a rubber, or a resin, etc. In some embodiments, the first conductive layer and the second conductive layer include conductive ink formed by mixing conductive particles with ink material. The conductive ink may be printed to form conductive patterns. In some embodiments, the first conductive layer and the second conductive layer include a conductive polymer material, a conductive gel, a liquid metal, etc. In some embodiments, the conductive polymer material includes, but is not limited to, polypyrrole, poly(3,4-ethylenedioxythiophene) polystyrene sulfonate (PEDOT:PSS), etc.

In some embodiments, the elastic conductive material includes an elastic material filled with conductive particles. Electrical conductivity of the first conductive layer and the second conductive layer may be adjusted by controlling a density of the conductive particles in the elastic material. In some embodiments, the elastomeric material includes silicone, rubber, resin, polydimethylsiloxane (PDMS), polyurethane, styrene-butadiene-styrene (SBS), etc. In some embodiments, the conductive particles include metal powder, carbon powder, carbon nanotubes, silver nanowires, carbon black, graphite powder, graphene, etc.

In some embodiments, the intermediate layer includes an elastically insulating material. At this point, each capacitive structure functions as a capacitive structure. For example, the elastically insulating material includes, but is not limited to, silicone, rubber, polydimethylsiloxane (PDMS), thermoplastic polyurethane (TPU), etc. In some embodiments, the intermediate layer includes a high-resistance elastic material, where a resistivity of the intermediate layer is greater than 1000 times that of the first conductive layer and the second conductive layer. At this point, each capacitive structure functions as a composite capacitive-resistive structure.

In some embodiments, a thickness of the first conductive layer and the second conductive layer of each capacitive structure ranges from 1 to 100 μm. In some embodiments, a width of the first conductive layer and the second conductive layer of each capacitive structure ranges from 0.5 to 10 mm. In some embodiments, a spacing between the first conductive layer 521 of the first capacitive structure 520 and the first conductive layer 531 of the second capacitive structure 530 ranges from 0.02 to 2 mm. In some embodiments, a spacing between the second conductive layer 522 of the first capacitive structure 520 and the second conductive layer 532 of the second capacitive structure 530 ranges from 0.02 to 2 mm. In some embodiments, a spacing between the first conductive layer 541 of the third capacitive structure 540 and the first conductive layer 551 of the fourth capacitive structure 550 ranges from 0.02 to 2 mm. In some embodiments, a spacing between the second conductive layer 542 of the third capacitive structure 540 and the second conductive layer 552 of the fourth capacitive structure 550 ranges from 0.02 to 2 mm.

In some embodiments, a conductivity of the first conductive layer is greater than a conductivity of the intermediate layer.

Conductivity is a parameter describing the ease of charge flow in a material. Since the conductivity of the first conductive layer is greater than the conductivity of the second conductive layer, the first conductive layer has better electrical conductivity. Therefore, the resistance of the first conductive layer is less than the resistance of the second conductive layer. It should be noted that, in the present disclosure, a resistance of a component refers to a resistance between two surfaces of the component spaced apart along the thickness direction of the flexible substrate 510. The resistance of the first conductive layer refers to a resistance between two surfaces of the first conductive layer spaced apart along the thickness direction of the flexible substrate 510. The resistance of the second conductive layer refers to a resistance between two surfaces of the second conductive layer spaced apart along the thickness direction of the flexible substrate 510. In some embodiments, the conductivity of the first conductive layer may be greater than 100 times that of the second conductive layer. In some embodiments, by setting a density of conductive particles filled in the elastic material of the first conductive layer to be greater than that of the second conductive layer, the conductivity of the first conductive layer may be made greater than that of the second conductive layer.

When a capacitive structure (e.g., the first capacitive structure 520, the second capacitive structure 530, the third capacitive structure 540, or the fourth capacitive structure 550) is a composite capacitive-resistive structure or a single capacitive structure, a resistance between two surfaces of the intermediate layer spaced apart along the thickness direction of the flexible substrate 510 should not be too small to facilitate subsequent measurement of the resistance and analysis of changes in the resistance. In some embodiments, the resistance between two surfaces of the intermediate layer spaced apart along the thickness direction of the flexible substrate 510 is greater than 0.8 MΩ. For example, when a capacitive structure (e.g., the first capacitive structure 520, the second capacitive structure 530, the third capacitive structure 540, or the fourth capacitive structure 550) is a composite capacitive-resistive structure, the resistance between two surfaces of the intermediate layer spaced apart along the thickness direction of the flexible substrate 510 may range from 0.8 MΩ to 550 GΩ. As another example, when a capacitive structure (e.g., the first capacitive structure 520, the second capacitive structure 530, the third capacitive structure 540, or the fourth capacitive structure 550) is a single capacitive structure, the resistance between two surfaces of the intermediate layer spaced apart along the thickness direction of the flexible substrate 510 may be larger, e.g., greater than 550 GΩ.

By setting the resistance between two surfaces of the intermediate layer spaced apart along the thickness direction of the flexible substrate 510 to be greater than 0.8 MΩ, the intermediate layer can conduct electricity while maintaining sufficiently high resistance, thereby ensuring accurate measurement of the resistance and analysis of the resistance changes. This enables accurate reflection of bending conditions of the second strain sensor 500.

In some embodiments, a relative permittivity of the intermediate layer is greater than 2. Relative permittivity is a physical parameter characterizing dielectric or polarizing properties of a material, defined as a ratio of capacitance of a capacitor with the material as dielectric to that with vacuum as dielectric at identical dimensions. Relative permittivity also represents a material's charge storage capacity and is termed relative capacitance. Materials with relative permittivity greater than 2 are polar substances, meaning the intermediate layer with relative permittivity greater than 2 has certain charge storage capacity. Therefore, the intermediate layer may be equivalently modeled as a parallel combination of resistive and capacitive elements.

In some embodiments, the relative permittivity of the intermediate layer may be greater than or equal to 4. Preferably, the relative permittivity of the intermediate layer is greater than 5. In some embodiments, the relative permittivity of the intermediate layer is greater than 10.

Setting the relative permittivity of the intermediate layer to be greater than 2 enables the intermediate layer to function as both a resistive element and a capacitive element. During bending of the second strain sensor 500, both capacitance and resistance of the intermediate layer change with deformation of the flexible substrate 510. Therefore, parameters related to capacitance and resistance of the intermediate layer may reflect bending conditions of the second strain sensor 500. In the embodiment, simultaneous existence of capacitance and resistance reflecting bending conditions significantly improves sensitivity and accuracy of the second strain sensor 500.

Additionally, by setting the resistance between two surfaces of the intermediate layer spaced apart along the thickness direction of the flexible substrate 510 to be greater than 0.8 MΩ, the intermediate layer exhibits excellent resistive and capacitive properties, ensuring sensitivity and accuracy of the second strain sensor 500.

In some embodiments, two capacitive structures on a same side surface of the flexible substrate 510 may share a first conductive layer, with second conductive layers of the two capacitive structures spaced apart along the short-axis direction of the flexible substrate 510. In some embodiments, more conductive layers (e.g., a third conductive layer) may be stacked along the thickness direction of each capacitive structure to improve sensitivity for detecting deformations in various degrees of freedom. For example, each capacitive structure includes a second conductive layer, a second intermediate layer, a third conductive layer, a first intermediate layer, and a first conductive layer stacked sequentially away from the flexible substrate 510 along the thickness direction. Parameters of the third conductive layer, the first conductive layer, and the second conductive layer are consistent. Parameters of the second intermediate layer, the first intermediate layer, and the intermediate layer described above are consistent.

In some embodiments, the glove system 400 may capture movement of a wrist joint. In the three-dimensional coordinate system shown in FIG. 3, the of movement the wrist joint (e.g., the wrist joint 230 in FIG. 2A) exhibits three degrees of freedom: bending, swinging, and rotation. Bending and swinging of the wrist joint are controlled by the wrist joint and cause deformation of the wrist joint. Rotation of the wrist joint is controlled by rotation of a user's forearm, causing overall rotation of the wrist joint.

In some embodiments, to capture movement in the three degrees of freedom of the wrist joint, the glove system 400 further includes two inertial sensors arranged on the glove body 410 in regions corresponding to opposite sides of the wrist joint. The processor 440 is configured to receive and process data collected by the inertial sensors. In some embodiments, the two inertial sensors are located on anterior and posterior sides of the wrist joint along an arm extension direction. The anterior side of the wrist joint faces toward a back of a hand and the posterior side of the wrist joint faces toward the forearm. For clarity, the two sides of the wrist joint may be understood as hand or forearm regions proximate to but not substantially deform with the deformation of the wrist joint deformation. For example, the glove system 400 includes a first inertial sensor 451 and a second inertial sensor 452. The first inertial sensor 451 and the second inertial sensor 452 on the glove body 410 at positions corresponding to hand positions 14 and 15 shown in FIG. 2B. The two inertial sensors respectively collect position information of opposite sides of the wrist joint, enabling identification of overall rotation, bending, and swinging movement of the wrist joint in three degrees of freedom.

In some embodiments, to capture movement in three degrees of freedom of the wrist joint, the glove system 400 may include an inertial sensor and a third strain sensor. The inertial sensor and the third strain sensor cooperate to capture the movement of the wrist joint. In some embodiments, the third strain sensor may include a multi-degree-of-freedom sensor for capturing deformations of the wrist joint in a plurality of degrees of freedom. Merely by way of example, the third strain sensor may be identical or similar to the second strain sensor, capturing deformations of the wrist joint in two or more degrees of freedom. As shown in FIG. 4, the inertial sensor and a third strain sensor 460 are arranged on the glove body 410. The third strain sensor 460 located at the wrist joint (i.e., on the glove body 410 at a position corresponding to a position between the hand position 14 and the hand position 15 shown in FIG. 2B). The inertial sensor is located outside the wrist joint region, e.g., at the anterior side or the posterior side of the wrist joint described above. In some embodiments, the inertial sensor may be located on either side of the wrist joint along the arm extension direction. For example, the inertial sensor is located on the glove body 410 at a position corresponding to hand position 14 or 15 shown in FIG. 2B, i.e., the inertial sensor is the first inertial sensor 451 or the second inertial sensor 452 shown in FIG. 4. The processor 440 is configured to receive and process data collected by the inertial sensor and the third strain sensor.

Using either two inertial sensors or a combination of an inertial sensor and a strain sensor enables the capture of multi-degree-of-freedom movement of the wrist joint. The users may flexibly select different combinations of sensor types according to different application scenarios to capture multi-degree-of-freedom movement.

Continuing with FIG. 4, in some embodiments, the glove body 410 includes a fabric wrapped around each finger of the hand and a fabric disposed between neighboring fingers. The fabric between the neighboring fingers connects finger bases (near metacarpophalangeal joints), potentially causing mutual pulling of the fabric at the neighboring metacarpophalangeal joints and interference between data from the second strain sensors at neighboring joints. To reduce such interference, the fabric between the neighboring fingers may include structures reducing or preventing pulling between the fabrics of the neighboring fingers, such that an elastic modulus of the fabric between the neighboring fingers is less than an elastic modulus of the fabric wrapped around each finger.

By arranging the structure between the fabrics of the neighboring fingers to reduce or avoid pulling between the fabrics at roots of the neighboring fingers during finger movement, such that the elastic modulus of the fabric between the neighboring fingers is less than the elastic modulus of the fabric wrapped around each finger, mutual interference between the second strain sensors at roots of different fingers can be reduced, ensuring that data collected by each second strain sensor accurately reflects deformation of the joint at the corresponding position.

In some embodiments, the structure that reduces pulling between the fabrics on neighboring fingers includes at least one of a slit structure, an elastic fabric structure, or a pleated structure. That is, the fabric disposed between the neighboring fingers include at least one of the slit structure, the elastic fabric structure, or the pleated structure. In some embodiments, as shown in FIG. 4, a slit structure 470 refers to a slit disposed in the glove fabric between the second strain sensors. The slit may be of any shape, such as a diamond, a rectangle, or the like. In some embodiments, an elasticity of the elastic fabric is greater than an elasticity of fabric in other regions of the glove body. In some embodiments, the elastic fabric structure or the pleated structure refers to an elastic fabric or a pleated fabric disposed between the second strain sensors, which can maintain the overall aesthetic appearance of the glove body fabric. In some embodiments, to further buffer pulling between the fabrics on the neighboring fingers, the slit structure, the elastic fabric structure, or the pleated structure extends along a finger length direction. In some embodiments, the pleated structure includes a plurality of pleats arranged along a direction between the neighboring fingers. In some embodiments, the elastic fabric structure or the pleated structure may coexist with the slit structure 470 and fill the slit structure 470, which reduces pulling between the fabrics at roots of the neighboring fingers while maintaining integrity of the glove body fabric.

In some embodiments, the processor is integrated on the glove body. For example, the processor may be located at a palm portion. As another example, as shown in FIG. 4, the processor 440 may be located at a back of the hand. The processor may be configured to: read data associated with the plurality of first strain sensors, the plurality of second strain sensors, the inertial sensor, and the third strain sensor; and determine a gesture of the user based on the data. For example, a function or machine learning may be used to establish a relationship between data and finger posture. Specifically, a raw dataset of sensor data and joint angles is obtained, based on the dataset a function is constructed or a machine learning model is trained. Thus, a mapping between the sensor data and the joint angles (or between the sensor data and the gestures) is generated. After determining the gesture of the user, it may be applied to a plurality of scenarios including sign language learning (action replication), voice generation for the deaf-mute, gesture manipulation methods, etc.

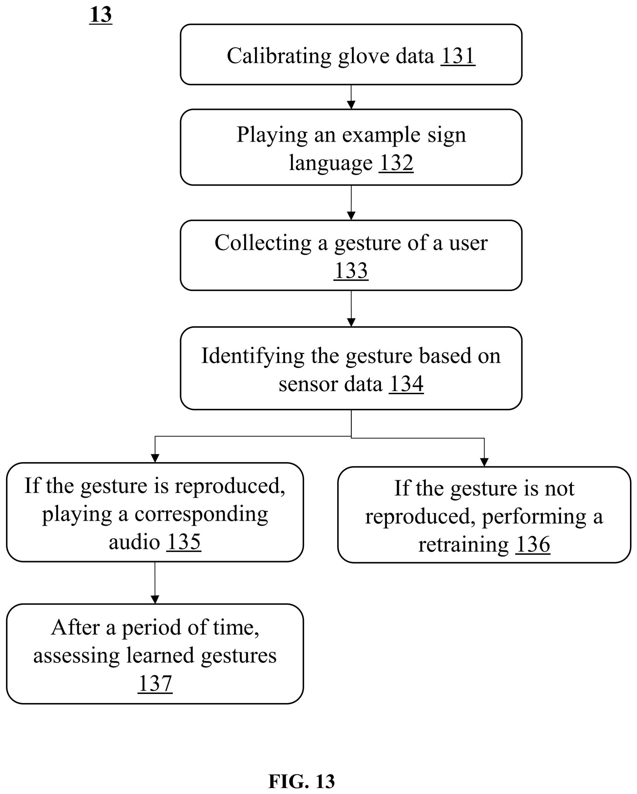

In some embodiments, after determining the gesture of the user, the processor may output a gesture representation corresponding to the gesture of the user on a display interface, and output feedback information based on a distance between the gesture representation and a preset gesture. The display interface is provided by an external device, e.g., a mobile phone screen or a computer screen. The feedback information may include a comparison result between the gesture representation and the preset gesture. The distance between gesture representation and the preset gesture may reflect a similarity level between the gesture of the user and the preset gesture. The smaller the distance is, the more similar the gesture of the user is to the preset gesture. In some embodiments, the distance may be obtained by mapping the gesture representation to the preset gesture and obtaining distances between corresponding points on the gesture representation and the preset gesture. The feedback information may be used to alert the user whether the gesture of the user matches the preset gesture or a similarity between the gesture of the user and the preset gesture. For example, the feedback information may be a similarity percentage between the gesture representation and the preset gesture. The processor's processing flow of outputting feedback information based on the gesture of the user and the distance to the preset gesture applies to a plurality of application scenarios, e.g., a gesture game application scenario shown in FIG. 12, or a sign language learning application scenario shown in FIG. 13.

FIG. 12 is a flowchart illustrating a process performed by a processor in accordance with some embodiments of the present disclosure.

As shown in FIG. 12, in some embodiments, in a gesture game scenario, the processing flow 12 of the processor includes the following operations.

In 121, an example animation may be played.

In some embodiments, the processor controls a display interface to display or play an animation including an example gesture. The example gesture is configured to guide the user in making the gesture. In some embodiments, the operation of playing the example animation may be triggered after the user wears a glove body, or may be triggered by the user.

In 122, glove data may be calibrated.

In some embodiments, the user wears the glove body and makes a gesture following the example gesture to perform data calibration for a glove system. The data calibration of the glove system includes a calibration of a deviation between a strain sensor position on the glove body and a corresponding joint position caused by wearing. Optionally, the example animation in operation 121 may include a gesture of a specific posture. When the user wears the glove body and imitates the gesture of the specific posture, the processor may calibrate data in a standard database using data collected at that time such that calibrated data more accurately reflects a finger posture of the user. In some embodiments, operation 122 is unnecessary or may be performed before operation 121.

In 123, a gesture of a player may be collected.

In some embodiments, after wearing the glove, the player makes a gesture causing strain sensors corresponding to joints of a hand of the player to generate strain data, such that the player's gesture is collected.

In 124, the gesture may be identified based on sensor data.

In some embodiments, the processor may reconstruct the gesture of the user based on strain data of the strain sensors on the glove body corresponding to each hand joint of the user. In some embodiments, the user may sequentially make a plurality of gestures, and the processor may sequentially identify the plurality of gestures based on the sensor data and store the gestures sequentially.

In 125, if the full set of gestures is reproduced, the game may be passed.

In some embodiments, the processor may compare the stored plurality of gestures of the user with the plurality of preset gestures in sequence, determine a distance between the plurality of gesture representations and the plurality of preset gestures, and output feedback information. If the distance between the plurality of gesture representations and the plurality of preset gestures is less than a preset threshold, it indicates that the full set of gesture reproduction is achieved, the processor outputs feedback information of "pass", and triggers the next game process. The distance between the plurality of gesture representations and the plurality of preset gestures may reflect the average similarity between the plurality of gestures of the user and the corresponding plurality of preset gestures. The smaller the distance, the higher the average similarity between the plurality of gestures and the one-to-one corresponding plurality of preset gestures. The preset threshold may correspond to, for example, an average match degree of 70% between the plurality of gesture representations and the plurality of preset gestures.

In 126, if the gesture is not reproduced, the game may be restarted.

In some embodiments, the processor may compare the stored plurality of gestures of the user with the plurality of preset gestures in sequence, determine the distance between the plurality of gesture representations and the plurality of preset gestures, and output feedback information. If the distance between the plurality of gesture representations and the plurality of preset gestures is greater than a preset threshold (e.g., corresponding to a 70% match degree between the plurality of gesture representations and the plurality of preset gestures), it indicates that the full set of gesture reproduction is not achieved, and the output feedback information is "gesture error".

In 127, a process of the player's gesture may be replayed, and a special effect animation may be played.

In some embodiments, the processor may control the display interface to sequentially display the gesture representations corresponding to the gesture of the users. In some embodiments, when the user achieves the full set of gesture reproduction, the processor may play back the player's gesture process and play special effect animations.

FIG. 13 is a processing flowchart illustrating a process performed by a processor according to some further embodiments of the present disclosure.

As shown in FIG. 13, in some embodiments, a processing flow 13 of the processor includes the following operation in a sign language learning application scenario.

In 131, glove data may be calibrated.

In some embodiments, the data calibration of the glove system includes a calibration of a deviation between the positions of strain sensors on the glove body and the corresponding joint positions caused by wearing. In some embodiments, a user wearing the glove body may make gestures to follow the gestures included in the calibration process shown on the display interface to perform the data calibration of the glove system.

In 132, an example sign language may be played.

In some embodiments, the processor controls the display interface to display or play the example sign language. The example sign language is configured to show the user standard sign language gestures, thereby guiding the user to learn the sign language. In some embodiments, the processing flow of playing the example sign language may be triggered automatically after the glove data is calibrated, or may be triggered by the user.

In 133, a gesture of the user may be collected.

In some embodiments, after the user wears the glove and makes the gesture, strain sensors on the glove corresponding to each hand joint of the user may be caused to generate strain data, so as to realize collection of the gesture.

In 134, the gesture may be identified based on sensor data.

In some embodiments, the processor may reconstruct the gesture of the user based on strain data of the strain sensors on the glove corresponding to each hand joint of the user. In some embodiments, the processor can display the reconstructed gesture of the users. In some embodiments, the processor can display the reconstructed gesture of the users on the display interface in the form of the gesture representations.

In 135, if the gesture is reproduced, a corresponding audio may be played.

In some embodiments, the processor may compare the gesture representation with an example sign language, determine the distance between the gesture representation and the example sign language, and output feedback. If the distance between the gesture representation and the example sign language is less than a preset threshold (e.g., corresponding to a 70% match degree between them), it means that the gesture reproduction is achieved. The output feedback information is to finish the training process and play the corresponding audio (e.g., "Congratulations on passing the training").

In 136, if the gesture is not reproduced, retraining may be performed.

In some embodiments, the processor may compare the gesture representation with an example sign language, determine a distance between the gesture representation and the example sign language, and output feedback. If the distance between the gesture representation and the example sign language is greater than a preset threshold (e.g., corresponding to a 70% match degree between them), it means the gesture reproduction is not achieved. The output feedback information is to restart the training process.

In 137, after a period of time, the learned gestures may be assessed.

In some embodiments, the processor may mark a preset gesture (i.e., example sign language) reproduced by the user and redisplay it after a period of time. For example, the processor may re-execute the training process for the preset gesture reproduced or reconstructed by the user in response to the user's instruction or automatically at intervals, so as to achieve repeated assessment.

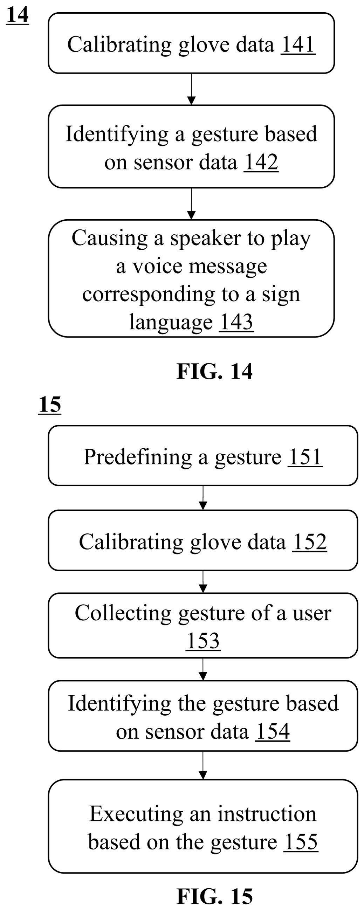

In some embodiments, after determining the gesture of the user, the processor may read a mapping relationship between gestures and voice packets, and play voice information based on the gesture of the user and the mapping relationship. The mapping relationship between the gestures and voice packets may be pre-constructed and stored in a database, or provided by a third party. The processor plays voice information based on the gesture of the users and the mapping relationship, which can be applied to various application scenarios, such as the application scenario of assisting deaf-mute individuals in voice production as shown in FIG. 14.

FIG. 14 is a flowchart illustrating a process performed by a processor according to some embodiments of the present disclosure.

As shown in FIG. 14, in some embodiments, in the application scenario of assisting deaf-mute individuals in voice production, the processing flow 14 of the processor includes the following operations.

In 141, glove data may be calibrated.

In some embodiments, data calibration of the glove system includes a calibration of a deviation between the positions of the strain sensors on the glove body and the positions of the corresponding joints caused by wearing. In some embodiments, the user wearing the glove body may perform the gestures included in the calibration process displayed on the display interface to perform data calibration of the glove system.

In 142, a gesture may be identified based on sensor data.

In some embodiments, when the user wears the glove and makes a sign language gesture, the strain sensors on the glove corresponding to each of the user's hand joints may generate strain data. The processor may reconstruct the gesture of the user based on the strain data from the strain sensors on the glove that correspond to each of the user's hand joints. In some embodiments, the user may make a plurality of gestures consecutively to express a complete sentence. The processor may recognize the plurality of gestures of the user consecutively based on the sensor data and store the gestures of the user in sequence.

In 143, a speaker may be caused to play a voice message corresponding to a sign language.

In some embodiments, the processor recognizes the gesture of the user and controls the speaker to play a voice message corresponding to the gesture of the user. The speaker may be provided by an external device. In some embodiments, the processor may recognize the gesture of the user and determine a distance between the gesture of the user and a preset gesture. For example, if the distance between the gesture of the user and the preset gesture is less than a preset threshold (e.g., corresponding to a 70% match between the gesture representation and the preset gesture), the processor may control the speaker to play the voice message; if the distance between the gesture of the user and the preset gesture is greater than the preset threshold (e.g., corresponding to a match lower than 70% between the gesture representation and the preset gesture), the voice message is not played, and the user is prompted to re-enter the gesture.

FIG. 15 is a flowchart illustrating a process performed by a processor according to some embodiments of the present disclosure.

In some embodiments, after determining the gesture of the user, the processor may read a mapping relationship between gestures and instructions, and execute an instruction based on the gesture of the user and the mapping relationship. The mapping relationship between the gestures and the instructions may be pre-constructed and stored in a database, or provided by a third party. The processor executes the instruction based on the gesture of the user and the mapping relationship, which may be applied to a plurality of application scenarios, such as the application scenario of the gesture manipulation shown in FIG. 15.

FIG. 15 is a flowchart illustrating a process performed by a processor according to some embodiments of the present disclosure.

As shown in FIG. 15, in some embodiments, in the application scenario of the gesture manipulation, the processing flow 15 of the processor includes the following operations.

In 151, a gesture may be predefined.

In some embodiments, the gesture may be predefined by the user; that is to say, the mapping relationship between the gestures and the instructions may be predefined by the user. In some embodiments, the user may predefine the mapping relationship between the gestures and the corresponding instructions for an external device (such as a game console, an IoT device). For example, the user may define the mapping relationship between a five-fingers-together gesture and the external device executing a screenshot instruction. For example, the user may define the mapping relationship between a five-fingers-stretching gesture and the external device executing a play instruction. As another example, the user may define the mapping relationship between a V-shaped gesture formed by the index finger and middle finger and the external device executing a photo-taking instruction.

In 152, glove data may be calibrated.

In some embodiments, data calibration of the glove system includes a calibration of a deviation between positions of the strain sensors on the glove body and positions of the corresponding joints caused by wearing. In some embodiments, data of the glove system may be calibrated at irregular intervals. The user wearing the glove body may make gestures following the gestures included in the calibration process displayed on the display interface to perform data calibration of the glove system.

In 153, the gesture of the user may be collected.

In some embodiments, after the user wears the glove and makes the gesture, the strain sensors on the glove corresponding to each hand joint of the user may be caused to generate strain data, so as to realize collection of the gesture of the user.

In 154, the gesture may be identified based on sensor data.

In some embodiments, the processor may reconstruct the gesture of the user based on strain data of the strain sensors on the glove corresponding to each hand joint of the user. In some embodiments, the processor may display the reconstructed gesture of the user on the display interface in the form of a gesture representation.

In 155, the instruction may be executed based on the gesture.

In some embodiments, the processor may identify the gesture of the user, and control an external device to execute the instruction corresponding to the gesture of the user according to the mapping relationship between the predefined gestures and the instructions by the user. For example, the user may enter a five-fingers-together gesture to control the external device to execute a screenshot instruction. For example, the user may input the gesture of five-fingers-stretching out to control the external device to execute the play instruction. As another example, the user may input the V-shaped gesture formed by the index finger and the middle finger to control the external device to execute the photo-taking instruction.

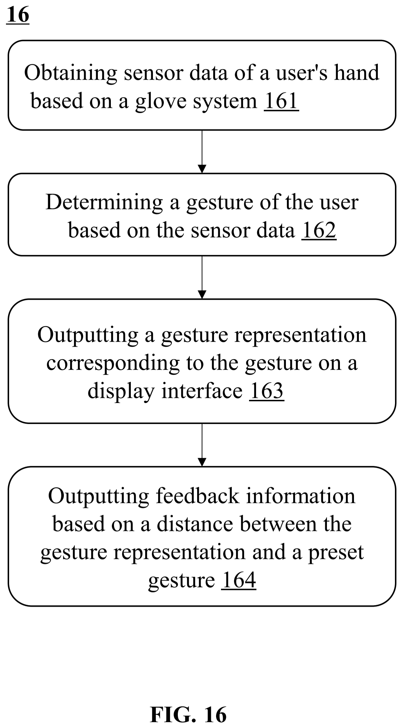

FIG. 16 is a flowchart illustrating a gesture reproduction method according to some embodiments of the present disclosure.

As shown in FIG. 16, in some embodiments, the gesture reproduction method 16 includes the following operations.

In 161, sensor data of a user's hand may be obtained based on a glove system.

In some embodiments, the glove system includes a glove body and a plurality of first strain sensors and a plurality of second strain sensors arranged on the glove body. When a user is wearing the glove body, each of the plurality of first strain sensors is located at an interphalangeal joint of the user and is configured to measure deformation of the interphalangeal joint in a single degree of freedom. Each of the plurality of second strain sensors is located at a metacarpophalangeal joint and is configured to measure deformation of the metacarpophalangeal joint in two degrees of freedom. The single degree of freedom and the two degrees of freedom both include a degree of freedom corresponding to a bending of the finger.

In some embodiments, the first strain sensors include single-degree-of-freedom sensors, such as single-axis sensors (e.g., inductive sensors, resistive bending sensors, or capacitive stretch sensors). In some embodiments, the second strain sensors include multi-degree-of-freedom sensors, such as multi-axis sensors (e.g., capacitive multi-axis motion sensors). The deformation of an interphalangeal joint corresponds to the single degree of freedom of bending. The deformation of the metacarpophalangeal joint corresponds to the two degrees of freedom of bending and swinging. Thus, each first strain sensor may measure deformation in the bending degree of freedom of one of the plurality of interphalangeal joints of the hand; that is, when any interphalangeal joint performs a bending movement, the first strain sensor may measure deformation of the glove body at a position corresponding to the interphalangeal joint. Each second strain sensor may measure deformation in the two degrees of freedom of bending and swinging of one of the plurality of metacarpophalangeal joints; that is, when any metacarpophalangeal joint performs a bending or swinging movement, the second strain sensor may measure deformation of the glove body at a position corresponding to the metacarpophalangeal joint. In total, the plurality of first strain sensors and the plurality of second strain sensors collectively provide deformation data reflecting at least 15 degrees of freedom of the user's fingers. For more content about the glove system, reference may be made to relevant parts above.

In 162, a gesture of the user may be determined based on the sensor data.

In some embodiments, when the user makes a gesture, it may cause strain sensors on the glove corresponding to each hand joint of the user to generate strain data, and the processor may reconstruct the gesture of the user based on the strain data of the strain sensors on the glove corresponding to each hand joint of the user.

In 163, a gesture representation corresponding to the gesture may be output on a display interface.

In some embodiments, after the gesture of the user is determined, the gesture representation corresponding to the gesture of the user may be output on the display interface. The gesture representation is a hand posture represented or simulated by means of points, lines, surfaces, or the like, or a combination thereof. For example, a plurality of joints of the user's hand may be represented by a plurality of points on the display interface, and bones between respective joints or a hand frame may be represented by connections between some of the points. As another example, a hand in a corresponding posture may be simulated based on the recognized gesture, and displayed on the display interface. The display interface is provided by an external device, e.g., a mobile phone screen, a computer screen, or the like. In some embodiments, the user may continuously make a plurality of gestures in sequence, and the processor may continuously recognize the plurality of gestures of the user based on the sensor data and display the gestures of the user in sequence.

In 164, feedback information may be output based on a distance between the gesture representation and a preset gesture.

In some embodiments, the processor may output the feedback information based on the distance between the gesture representation and the preset gesture. The feedback information includes a comparison result between the gesture representation and the preset gesture, or the like. The distance between the gesture representation and the preset gesture reflects the degree of similarity between the gesture of the user and the preset gesture. The smaller the distance, the higher the degree of similarity between the gesture of the user and the preset gesture. In some embodiments, the distance may be obtained by first mapping the gesture representation and the preset gesture, and further obtaining distances between corresponding points on the gesture representation and the preset gesture. The feedback information is used to remind the user whether the gesture is the same as the preset gesture or the degree of similarity between the gesture and the preset gesture. The processing flow in which the processor outputs the feedback information based on the gesture of the user and the distance from the preset gesture may be applied to a plurality of application scenarios, such as a gesture game application scenario, a sign language learning application scenario, or the like.

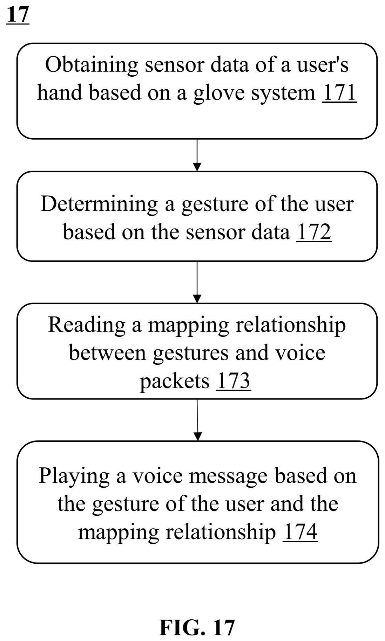

FIG. 17 is a flowchart illustrating a voice playback method according to some embodiments of the present disclosure.

As shown in FIG. 17, in some embodiments, a voice playback method 17 includes the following operations.

In 171, sensor data of a user's hand may be obtained based on a glove system.

In some embodiments, the glove system includes a glove body and a plurality of first strain sensors and a plurality of second strain sensors arranged on the glove body. When a user is wearing the glove body, each of the plurality of first strain sensors is located at an interphalangeal joint of the user and is configured to measure deformation of the interphalangeal joint in a single degree of freedom. Each of the plurality of second strain sensors is located at a metacarpophalangeal joint and is configured to measure deformation of the metacarpophalangeal joint in two degrees of freedom. The single degree of freedom and the two degrees of freedom both include a degree of freedom corresponding to a bending of the finger.

In some embodiments, the first strain sensor includes a single-degree-of-freedom sensor, such as a single-axis sensor (e.g., an inductive sensor, a resistive bending sensor, or a capacitive stretch sensor). In some embodiments, the second strain sensor includes a multi-degree-of-freedom sensor, such as a multi-axis sensor (e.g., a capacitive multi-axis motion sensor). The deformation of the interphalangeal joint corresponds to the single degree of freedom of bending. The deformation of the metacarpophalangeal joint corresponds to the two degrees of freedom of bending and swinging. Thus, each first strain sensor may measure deformation in the bending degree of freedom of one of the plurality of interphalangeal joints of the hand; that is, when any interphalangeal joint performs a bending motion, the first strain sensor may measure deformation of the glove body at a position corresponding to the interphalangeal joint. Each second strain sensor may measure deformation in the two degrees of freedom of bending and swinging of one of the plurality of metacarpophalangeal joints; that is, when any metacarpophalangeal joint performs a bending or swinging motion, the second strain sensor may measure deformation of the glove body at a position corresponding to the metacarpophalangeal joint. In total, the plurality of first strain sensors and the plurality of second strain sensors collectively provide deformation data reflecting at least 15 degrees of freedom of the user's fingers. For more content about the glove system, reference may be made to relevant parts above.

In 172, a gesture of the user may be determined based on the sensor data.

In some embodiments, when the user makes a gesture, it may cause strain sensors on the glove corresponding to each hand joint of the user to generate strain data, and the processor may reconstruct the gesture of the user based on the strain data of the strain sensors on the glove corresponding to each hand joint of the user.

In 173, a mapping relationship between the gestures and voice packets may be read.

In some embodiments, the mapping relationship between the gestures and the voice packets may be predefined by the user. For example, the user may define a mapping relationship between a five-fingers-together gesture and a voice "stop". For example, the user may define a mapping relationship between a five-fingers-together a five-fingers-stretching gesture and a voice "hey". As another example, the user may define a mapping relationship between a V-shaped gesture formed by the index finger and the middle finger and a voice "take a photo". In some embodiments, the mapping relationship between the gestures and the voice packets may also be established by a sign language database, i.e., the sign language database includes the mapping relationship between the sign language gestures and the corresponding spoken vocabulary.

In 174, a voice message may be played based on the gesture of the user and the mapping relationship.

In some embodiments, the processor recognizes the gesture of the user and, according to the mapping relationship between the gestures and the voice packets, controls an external device (e.g., a loudspeaker) to play voice information. For example, the user may input a five-fingers-together to control the external device to output the voice "stop". For example, the user may input a five-fingers-stretching gesture to control the external device to output the voice "hey". As another example, the user may input a V-shaped gesture formed by the index finger and the middle finger to control the external device to output the voice "take a photo". As yet another example, the user may input a sign language gesture to control the external device to output the voice corresponding to the sign language.

For more content about the voice playback method, reference may be made to FIG. 14 and related descriptions thereof.

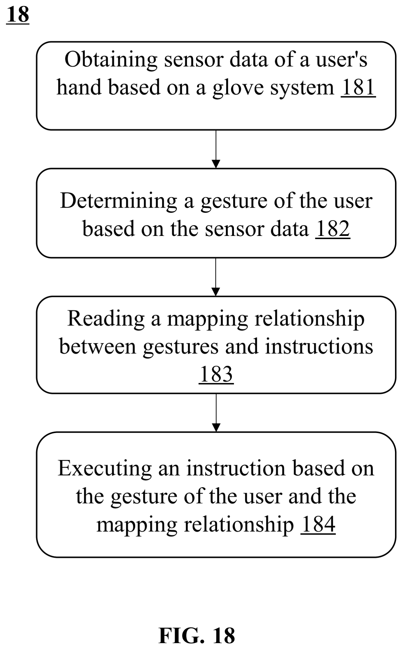

FIG. 18 is a flowchart illustrating a gesture manipulation method according to some embodiments of the present disclosure.

As shown in FIG. 18, in some embodiments, a gesture manipulation method 18 includes the following operations.

In 181, sensor data of a user's hand may be obtained based on a glove system.

In some embodiments, a glove system includes a glove body and a plurality of first strain sensors and a plurality of second strain sensors arranged on the glove body. When a user is wearing the glove body, each of the plurality of first strain sensors is located at an interphalangeal joint of the user and is configured to measure deformation of the interphalangeal joint in a single degree of freedom. Each of the plurality of second strain sensors is located at a metacarpophalangeal joint and is configured to measure deformation of the metacarpophalangeal joint in two degrees of freedom. The single degree of freedom and the two degrees of freedom both include a degree of freedom corresponding to a bending of the finger.

In some embodiments, the first strain sensor includes a single-degree-of-freedom sensor, such as a single-axis sensor (e.g., an inductive sensor, a resistive bending sensor, or a capacitive stretch sensor). In some embodiments, the second strain sensor includes a multi-degree-of-freedom sensor, such as a multi-axis sensor (e.g., capacitive multi-axis motion sensors). The interphalangeal joint corresponds to the single degree of freedom of bending. The metacarpophalangeal joint corresponds to the two degrees of freedom of bending and swinging. Thus, each first strain sensor may measure deformation in the bending degree of freedom of one of the 9 interphalangeal joints of the hand; that is, when any interphalangeal joint performs a bending movement, the first strain sensor may measure deformation of the glove body at a position corresponding to the interphalangeal joint. Each second strain sensor may correspondingly measure deformation in the two degrees of freedom of bending and swinging of one of the 5 metacarpophalangeal joints; that is, when any metacarpophalangeal joint performs a bending or swinging movement, the second strain sensor may measure deformation of the glove body at a position corresponding to the metacarpophalangeal joint. In total, the plurality of first strain sensors and the plurality of second strain sensors collectively provide deformation data reflecting at least 15 degrees of freedom of the user's fingers. For more content about the glove system, reference may be made to relevant parts above.

In 182, a gesture of the user may be determined based on the sensor data.

In some embodiments, when the user makes the gesture after wearing the glove, it may cause strain sensors on the glove corresponding to each hand joint of the user to generate strain data. The processor may reconstruct the gesture of the user based on the strain data of the strain sensors on the glove corresponding to each hand joint of the user. In some embodiments, the processor may display the reconstructed gesture of the user on a display interface in the form of a gesture representation.

In 183, a mapping relationship between gestures and instructions may be read.

In some embodiments, the mapping relationship between the gestures and the execution instructions may be predefined by the user. In some embodiments, the user may predefine the mapping relationship between the gestures and the corresponding instructions for an external device (e.g., a game console). For example, the user may define a mapping relationship between a five-fingers-together gesture and the external device executing a screenshot instruction. For example, the user may define a mapping relationship between a five-fingers-stretching gesture and the external device executing a play instruction. As another example, the user may define a mapping relationship between a V-shaped gesture formed by the index finger and the middle finger and the external device executing a photo-taking instruction. In some embodiments, the mapping relationship between the gesture and the execution instruction may also be defined by manufacturers.

In 184, an instruction may be executed based on the gesture of the user and the mapping relationship.

In some embodiments, the processor recognizes the gesture of the user and, according to the user-predefined mapping relationship between the gestures and the instructions, controls the external device to execute the instruction corresponding to the gesture of the user. For example, the user may input the five-fingers-together to control the external device to execute the screenshot instruction. For example, the user may input the five-fingers-stretching gesture to control the external device to execute the play instruction. As another example, the user may input the V-shaped gesture formed by the index finger and the middle finger to control the external device to execute the photo-taking instruction.

For more content about the gesture manipulation method, reference may be made to FIG. 15 and related descriptions thereof.

The foregoing has described the basic concepts. Clearly, for those skilled in the art, the above detailed disclosure is merely provided as an example and does not constitute a limitation of the present disclosure. Although not explicitly stated herein, those skilled in the art may make various modifications, improvements, and adaptations to the present disclosure. Such modifications, improvements, and adaptations are suggested in the present disclosure and therefore fall within the spirit and scope of the exemplary embodiments of the present disclosure.

Meanwhile, the present disclosure uses specific terms to describe embodiments of the present disclosure. Expressions such as "one embodiment," "an embodiment," and/or "some embodiments" refer to a certain feature, structure, or characteristic associated with at least one embodiment of the present disclosure. Therefore, it should be emphasized and noted that the repeated references to "an embodiment," "one embodiment," or "an alternative embodiment" in different parts of the present disclosure do not necessarily refer to the same embodiment. In addition, certain features, structures, or characteristics in one or more embodiments of the present disclosure may be appropriately combined. Furthermore, unless explicitly stated in the claims, the order of processing elements and sequences, the use of numerals or letters, or the use of other names in the present disclosure are not intended to limit the sequence of processes and methods described herein.

Furthermore, unless explicitly stated in the claims, the order of processing elements and sequences, the use of numerals or letters, or the use of other names in the present disclosure are not intended to limit the sequence of processes and methods described herein. Although some presently considered useful embodiments have been discussed above by way of various examples, it should be understood that such details are provided merely for illustrative purposes. The appended claims are not limited to the disclosed embodiments; instead, the claims are intended to cover all modifications and equivalent combinations that fall within the spirit and scope of the embodiments of the present disclosure. For example, although the system components described above may be implemented through hardware devices, the same may also be achieved solely through software solutions, such as installing the described system on existing servers or mobile devices.

Similarly, it should be noted that in order to simplify the presentation of the disclosure of this specification, and thereby aid in the understanding of one or more embodiments of the invention, the foregoing descriptions of embodiments of this specification sometimes combine a variety of features into a single embodiment, accompanying drawings, or the description thereof. description thereof. However, this method of disclosure does not imply that the objects of the present specification require more features than those mentioned in the claims. Rather, claimed subject matter may lie in less than all features of a single foregoing disclosed embodiment.

Numbers describing the number of compositions, attributes are used in some embodiments, and it should be understood that such numbers used in the description of embodiments, in some examples, use the modifiers "about ", "approximately", or "generally" is used in some examples. Unless otherwise noted, the terms "about," "approximate," or "approximately" indicates that a $\pm20\%$ variation in the stated number is allowed. Correspondingly, in some embodiments, the numerical parameters used in the specification and claims are approximations, which approximations are subject to change depending on the desired characteristics of the individual embodiment. In some embodiments, the numerical parameters should take into account the specified number of valid digits and use a general digit retention method. While the numerical domains and parameters used to confirm the breadth of their ranges in some embodiments of this specification are approximations, in specific embodiments such values are set to be as precise as possible within a feasible range.

For each of the patents, patent applications, patent application disclosures, and other materials cited in this specification, such as articles, books, specification sheets, publications, documents, and the like, the entire contents thereof are hereby incorporated herein by reference. Application history documents that are inconsistent with or conflict with the contents of this specification are excluded, as are documents (currently or hereafter appended to this specification) that limit the broadest scope of the claims of this specification. It should be noted that in the event of any inconsistency or conflict between the descriptions, definitions, and/or use of terms in the materials appended to this specification and those set forth herein, the descriptions, definitions and/or use of terms in this specification shall control. use shall prevail.

Finally, it should be understood that the embodiments described in this specification are used only to illustrate the principles of the embodiments of this specification. Other deformations may also fall within the scope of this specification. As such, alternative configurations of embodiments of the present specification may be viewed as consistent with the teachings of the present specification as an example, not as a limitation. Correspondingly, the embodiments of the present specification are not limited to the embodiments expressly presented and described herein.

**Claims**

1. A glove system, comprising:

   a glove body; and
   a plurality of first strain sensors and a plurality of second strain sensors arranged on the glove body, wherein when a user is wearing the glove body, each of the plurality of first strain sensors is located at an interphalangeal joint of a finger of the user and is configured to measure deformation of the interphalangeal joint in a single degree of freedom, and each of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger.

2. The glove system according to claim 1, wherein the plurality of first strain sensors and the plurality of second strain sensors collectively provide data reflecting deformation of the fingers of the user in no less than 15 degrees of freedom.

3. The glove system according to claim 1, further comprising two inertial sensors, wherein when the user is wearing the glove body, the two inertial sensors are respectively located on two sides of a wrist joint of the user and are configured to jointly measure movement of the wrist joint in three degrees of freedom.

4. The glove system according to claim 1, further comprising a third strain sensor disposed at the wrist joint of the user and an inertial sensor disposed outside the wrist joint.

5. The glove system according to claim 1, wherein a first strain sensor of the plurality of first strain sensors includes an inductive sensor, and an inductance of the inductive sensor varies with the deformation of the interphalangeal joint.

6. The glove system according to claim 1, wherein a second strain sensor of the plurality of second strain sensors includes a plurality of capacitive structures, capacitances of the plurality of capacitive structures vary with the deformation of the metacarpophalangeal joint in each degree of freedom.

7. The glove system according to claim 6, wherein the second strain sensor includes:

   a flexible substrate;
   a first capacitive structure and a second capacitive structure, each of the first capacitive structure and the second capacitive structure includes a multilayer structure arranged on a same side surface of the flexible substrate in a thickness direction, and layers of each of the multilayer structures are stacked along the thickness direction.

8. The glove system according to claim 1, wherein the glove body includes a fabric wrapped around each finger of the hand and a fabric disposed between neighboring fingers, an elastic modulus of the fabric between the neighboring fingers is less than an elastic modulus of the fabric wrapped around each finger.

9. The glove system according to claim 8, wherein the fabric disposed between the neighboring fingers includes at least one of a slit structure, an elastic fabric structure, or a pleated structure.

10. The glove system according to claim 1, wherein the plurality of first strain sensors are arranged only at the interphalangeal joints of the fingers that are closest to the metacarpophalangeal joints.

11. The glove system according to claim 1, further comprising a processor configured to:

read data associated with the plurality of first strain sensors and the plurality of second strain sensors; and determine a gesture of the user based on the data.

12. The glove system according to claim 11, wherein the processor is configured to:

output a gesture representation corresponding to the gesture of the user on a display interface ; and output feedback information based on a distance between the gesture representation and a preset gesture.

13. The glove system according to claim 11, wherein the processor is configured to:

read a mapping relationship between gestures and voice packets; and play a voice message based on the gesture of the user and the mapping relationship.

14. The glove system according to claim 11, wherein the processor is configured to:

read a mapping relationship between gestures and instructions; and execute an instruction based on the gesture of the user and the mapping relationship.

15. A gesture reproduction method, comprising:

obtaining sensor data of a hand of a user based on a glove system, wherein the glove system includes a glove body, and a plurality of first strain sensors and a plurality of second strain sensors arranged on the glove body, wherein when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger; determining a gesture of the user based on the sensor data; outputting a gesture representation corresponding to the gesture on a display interface; and outputting feedback information based on a distance between the gesture representation and a preset gesture.

16. The gesture reproduction method according to claim 15, wherein the plurality of first strain sensors and the plurality of second strain sensors collectively provide data reflecting deformation of the fingers of the user in no less than 15 degrees of freedom.

17. A voice playback method, comprising:

obtaining sensor data of a hand of a user based on a glove system, wherein the glove system includes a glove body, and a plurality of first strain sensors and a plurality of second strain sensors arranged on the glove body, wherein when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger; determining a gesture of the user based on the sensor data; reading a mapping relationship between gestures and voice packets; and playing a voice message based on the gesture of the user and the mapping relationship.

18. The voice playback method according to claim 17, wherein the plurality of first strain sensors and the plurality of second strain sensors collectively provide data reflecting deformation of the fingers of the user in no less than 15 degrees of freedom.

19. A gesture manipulation method, comprising:

obtaining sensor data of a hand of a user based on a glove system, wherein the glove system includes a glove body, and a plurality of first strain sensors and a plurality of second strain sensors arranged on the glove body, wherein when the user is wearing the glove body, each first strain sensor of the plurality of first strain sensors is located at an interphalangeal joint of a finger of the user and is configured to measure deformation of the interphalangeal joint in a single degree of freedom, and each second strain sensor of the plurality of second strain sensors is located at a metacarpophalangeal joint of the finger and is configured to measure deformation of the metacarpophalangeal joint in two degrees of freedom, the single degree of freedom and the two degrees of freedom both including a degree of freedom corresponding to a bending of the finger;

determining a gesture of the user based on the sensor data;

reading a mapping relationship between gestures and instructions; and

executing an instruction based on the gesture of the user and the mapping relationship.

20. The gesture manipulation method according to claim 19, wherein the plurality of first strain sensors and the plurality of second strain sensors collectively provide data reflecting deformation of the fingers of the user in no less than 15 degrees of freedom.

**100**

Glove body <u>110</u>

First strain sensor <u>120</u>

Second strain sensor <u>130</u>

Processor<u>140</u>

**FIG. 1**

**FIG. 2A**

**FIG. 2B**

**FIG. 3**

**FIG. 4**

**500**

**FIG. 5**

**500**

**FIG. 6**

**500**

S1

A

B

S2

530

510

Z

Y

X

520

540

550

**FIG. 7A**

S1

520

530

510

S2

540

550

**FIG. 7B**

**FIG. 8A**

**FIG. 8B**

**FIG. 9**

**FIG. 10**

**FIG. 11**

12

Playing an example animation 121

Calibrating glove data 122

Collecting player's gesture 123

Identifying the gesture based on sensor data 124

If full set of gestures is reproduced, passing the game 125

If the gesture is not reproduced, restarting the game 126

Replaying a process of the player's gesture and playing a special effect animation 127

**FIG. 12**

**13**

Calibrating glove data 131

↓

Playing an example sign language 132

↓

Collecting a gesture of a user 133

↓

Identifying the gesture based on sensor data 134

If the gesture is reproduced, playing a corresponding audio 135

If the gesture is not reproduced, performing a retraining 136

↓

After a period of time, assessing learned gestures 137

**FIG. 13**

**14**

Calibrating glove data 141

↓

Identifying a gesture based on sensor data 142

↓

Causing a speaker to play a voice message corresponding to a sign language 143

**FIG. 14**

**15**

Predefining a gesture 151

↓

Calibrating glove data 152

↓

Collecting gesture of a user 153

↓

Identifying the gesture based on sensor data 154

↓

Executing an instruction based on the gesture 155

**FIG. 15**

**16**

```
┌─────────────────────────────────────┐
│  Obtaining sensor data of a user's   │
│  hand                                │
│  based on a glove system 161         │
└─────────────────────────────────────┘
                  │
                  ▼
┌─────────────────────────────────────┐
│  Determining a gesture of the user   │
│  based on the sensor data 162        │
└─────────────────────────────────────┘
                  │
                  ▼
┌─────────────────────────────────────┐
│  Outputting a gesture representation │
│  corresponding to the gesture on a   │
│  display interface 163               │
└─────────────────────────────────────┘
                  │
                  ▼
┌─────────────────────────────────────┐
│  Outputting feedback information     │
│  based on a distance between the     │
│  gesture representation and a preset │
│  gesture 164                         │
└─────────────────────────────────────┘
```

**FIG. 16**

<u>**17**</u>

```
┌─────────────────────────────────┐
│  Obtaining sensor data of a user's │
│  hand based on a glove system 171  │
└─────────────────────────────────┘
                 │
                 ▼
┌─────────────────────────────────┐
│  Determining a gesture of the user │
│  based on the sensor data 172      │
└─────────────────────────────────┘
                 │
                 ▼
┌─────────────────────────────────┐
│  Reading a mapping relationship   │
│  between gestures and voice        │
│  packets 173                       │
└─────────────────────────────────┘
                 │
                 ▼
┌─────────────────────────────────┐
│  Playing a voice message based on │
│  the gesture of the user and the   │
│  mapping relationship 174          │
└─────────────────────────────────┘
```

**FIG. 17**

**18**

Obtaining sensor data of a user's hand based on a glove system 181

Determining a gesture of the user based on the sensor data 182

Reading a mapping relationship between gestures and instructions 183

Executing an instruction based on the gesture of the user and the mapping relationship 184

**FIG. 18**

## INTERNATIONAL SEARCH REPORT

| International application No. |
| --- |
| **PCT/CN2024/099228** |

**A. CLASSIFICATION OF SUBJECT MATTER**

G06F3/01(2006.01)i; A41D19/00(2006.01)i; G01B7/16(2006.01)i

According to International Patent Classification (IPC) or to both national classification and IPC

**B. FIELDS SEARCHED**

Minimum documentation searched (classification system followed by classification symbols)

IPC: G06F3/-; B25J3/-; B25J13/-; G01B7/-; A41D19/-; A61B5/-

Documentation searched other than minimum documentation to the extent that such documents are included in the fields searched

Electronic data base consulted during the international search (name of data base and, where practicable, search terms used)

CNABS, CNTXT, CNKI, ENTXTC, ENTXT, DWPI, WPABS, WPABSC, EPTXT, JPTXT, USTXT, WOTXT: 智能手套, 数据手套, 柔性, 弯曲, 应变, 形变, 传感器, 关节, 手势, 手部, 手指, 手掌, 手腕, 自由度, 维度, 电容, 多层, 堆叠, 叠置, 并排, 同一侧, glove, flexible, bend+, deform+, strain, sensor, joint, finger, hand, pose, gesture, freedom, capacitor, multi+, layer, stack

**C. DOCUMENTS CONSIDERED TO BE RELEVANT**

| Category* | Citation of document, with indication, where appropriate, of the relevant passages | Relevant to claim No. |
| --- | --- | --- |
| X | CN 107825393 A (BEIJING UNIVERSITY OF TECHNOLOGY) 23 March 2018 (2018-03-23) description, paragraphs [0030]-[0049], and figures 1-5 | 1-2, 5-6, 8-20 |
| Y | CN 107825393 A (BEIJING UNIVERSITY OF TECHNOLOGY) 23 March 2018 (2018-03-23) description, paragraphs [0030]-[0049], and figures 1-5 | 3-4, 7 |
| Y | CN 109407836 A (ROTEX INC.) 01 March 2019 (2019-03-01) description, paragraphs [0043]-[0047] and [0059]-[0073], and figures 1-7 | 3-4 |
| Y | CN 115371853 A (QINGDAO GOERTEK INTELLIGENT SENSOR CO., LTD.) 22 November 2022 (2022-11-22) description, paragraphs [0044]-[0046], and figures 1-3 | 7 |
| A | CN 109901708 A (BEIJING UNIVERSITY OF POSTS AND TELECOMMUNICATIONS) 18 June 2019 (2019-06-18) entire document | 1-20 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 August 2024** | **27 August 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/099228**

### C. DOCUMENTS CONSIDERED TO BE RELEVANT

| Category* | Citation of document, with indication, where appropriate, of the relevant passages | Relevant to claim No. |
|---|---|---|
| A | CN 111722723 A (BEIJING UNIVERSITY OF CHEMICAL TECHNOLOGY) 29 September 2020 (2020-09-29) entire document | 1-20 |
| A | US 2017176267 A1 (OCULUS VR, L. L. C.) 22 June 2017 (2017-06-22) entire document | 1-20 |
| A | US 2020309508 A1 (SONY CORP.) 01 October 2020 (2020-10-01) entire document | 1-20 |
| A | WO 2019173678 A1 (SIEMENS AG et al.) 12 September 2019 (2019-09-12) entire document | 1-20 |

Form PCT/ISA/210 (second sheet) (July 2022)

INTERNATIONAL SEARCH REPORT
Information on patent family members

International application No.

**PCT/CN2024/099228**

| Patent document cited in search report | | | Publication date (day/month/year) | Patent family member(s) | | | Publication date (day/month/year) |
|---|---|---|---|---|---|---|---|
| CN | 107825393 | A | 23 March 2018 | None | | | |
| CN | 109407836 | A | 01 March 2019 | WO | 2020073891 | A1 | 16 April 2020 |
| | | | | CN | 209216040 | U | 06 August 2019 |
| CN | 115371853 | A | 22 November 2022 | None | | | |
| CN | 109901708 | A | 18 June 2019 | None | | | |
| CN | 111722723 | A | 29 September 2020 | CN | 111722723 | B | 13 July 2021 |
| US | 2017176267 | A1 | 22 June 2017 | US | 10197459 | B2 | 05 February 2019 |
| | | | | US | 10458864 | B1 | 29 October 2019 |
| US | 2020309508 | A1 | 01 October 2020 | US | 11125547 | B2 | 21 September 2021 |
| | | | | JPWO | 2018003527 | A1 | 18 April 2019 |
| | | | | WO | 2018003527 | A1 | 04 January 2018 |
| WO | 2019173678 | A1 | 12 September 2019 | None | | | |

Form PCT/ISA/210 (patent family annex) (July 2022)

**REFERENCES CITED IN THE DESCRIPTION**

**Patent documents cited in the description**

- CN 2023124295 W